# FASCICULE DE BREVET EUROPEEN

(11) **EP 2 435 835 B1**
(45) Date de publication et mention de la délivrance du brevet: **28.08.2013**
(21) Numéro de dépôt: 10728802.9
(22) Date de dépôt: 21.05.2010
(51) Int. Cl.: G01N 33/68

(54) **NOUVEAU PROCEDE DE QUANTIFICATION DE PROTEINES PAR SPECTROMETRIE DE MASSE**
NEUES VERFAHREN ZUR QUANTIFIZIERUNG VON PROTEINEN MITTELS MASSENSPEKTROMETRIE
NOVEL METHOD FOR QUANTIFYING PROTEINS BY MASS SPECTROMETRY

(30) Priorité: 29.05.2009 FR 0953576
(43) Date de publication de la demande: 04.04.2012
(73) Titulaire: Biomérieux, 69280 Marcy l'Étoile (FR); Université Claude Bernard Lyon I, 69622 Villeurbanne Cedex (FR); Centre National de la Recherche Scientifique, 75794 Paris Cedex 16 (FR)
(72) Inventeur: LEMOINE, Jérôme, 69480 Lucenay (FR); SALVADOR, Arnaud, F-38080 L'ile d'Abeau (FR); CHARRIER, Jean-Philippe, F-69160 Tassin La Demi-Lune (FR); FORTIN, Tanguy, 69006 Lyon (FR)
(74) Mandataire: Sarlin, Laure V.
(86) Numéro de dépôt international: PCT/FR2010/050991
(87) Numéro de publication internationale: WO 2010/136706

(56) Documents cités:
- WO-A1-2009/141310
- HAN BOMIE ET AL: "Proteomics: from hypothesis to quantitative assay on a single platform. Guidelines for developing MRM assays using ion trap mass spectrometers" BRIEFINGS IN FUNCTIONAL GENOMICS & PROTEOMICS, vol. 7, no. 5, septembre 2008 (2008-09), pages 340-354, XP009128978 ISSN: 1473-9550
- IZRAEL-TOMASEVIC ANITA ET AL: "Targeting Interferon Alpha Subtypes in Serum: A Comparison of Analytical Approaches to the Detection and Quantitation of Proteins in Complex Biological Matrices" JOURNAL OF PROTEOME RESEARCH, vol. 8, no. 6, juin 2009 (2009-06), pages 3132-3140, XP002566819 ISSN: 1535-3893
- FORTIN T ET AL: "Multiple reaction monitoring cubed for protein quantification at the low nanogram/milliliter level in nondepleted human serum." ANALYTICAL CHEMISTRY 15 NOV 2009, vol. 81, no. 22, 15 novembre 2009 (2009-11-15), pages 9343-9352, XP002566821 ISSN: 1520-6882

## Description

La présente invention concerne le domaine technique du dosage quantitatif de protéines. En particulier, la présente invention concerne un nouveau procédé de dosage quantitatif de protéine par spectrométrie de masse.

De nombreuses techniques de dosage quantitatif de protéines ont été développées, le dosage de protéines dans des fluides complexes comme des échantillons sanguins (sérum, plasma) de patients étant d'une importance capitale en diagnostic. Les dosages ELISA (dosage d'immunosorption liée à enzyme, de l'anglais « Enzyme-linked immunosorbent assay ») sont les plus utilisés à ce jour. Parmi les différentes techniques ELISA connues, la réaction de type sandwich est la plus utilisée. Elle nécessite deux anticorps de la protéine d'intérêt, l'un étant lié à l'enzyme. Plus récemment, le dosage quantitatif de protéines, *via* leurs peptides protéotypiques, par des techniques de spectrométrie de masse telles que la SRM de l'anglais « Selected Reaction Monitoring » ou la MRM de l'anglais « Multiple Reaction Monitoring » lorsque plusieurs dosages SRM sont réalisés simultanément, a été validé, dans des fluides complexes, par la demanderesse (T. Fortin et al., MCP, 2008 E-pub) et d'autres (L. Anderson & C. Hunter, MCP, 2006, 573-588 ; H. Zhang et al., MCP, 2007, 64-71). En amont du dosage par spectrométrie de masse, les protéines à doser sont digérées, grâce à une enzyme, pour fragmenter les protéines en peptides. Les peptides spécifiques de la protéine, nommés peptides protéotypiques, sont alors dosés par spectrométrie de masse.

L'avantage des dosages par SRM ou MRM par rapport aux dosages ELISA, est une réduction considérable du coût et de la durée de développement du dosage, notamment si des anticorps nécessaires au dosage ELISA doivent être développés. De telles techniques par spectrométrie de masse apparaissent donc comme des méthodes de choix pour le dosage de protéines, et permettraient, par exemple de valider, plus simplement et plus rapidement, l'intérêt clinique du dosage des nombreuses protéines identifiées comme marqueurs potentiels par les recherches en analyse protéomique (S. Carr & L. Anderson, Clin. Cem. 2008, 1749-1752).

Dans le cas d'un dosage MRM, dans un spectromètre de masse de type triple quadripôle, particulièrement adapté au mode MRM, les peptides protéotypiques sont quantifiés selon le principe détaillé ci-après. Tout d'abord, l'échantillon contenant les peptides à doser est introduit dans une source d'ionisation, où les peptides sont ionisés à l'état gazeux et transformés en ions dits moléculaires qui correspondent aux peptides initiaux avec un, deux, voire trois protons supplémentaires et sont donc porteurs de une, deux, voire trois charges. On obtient par exemple, grâce à une source de type électrospray, une ionisation des peptides, tout en les faisant passer d'un état liquide à un état gazeux (Gaskell, Electrospray: principles and practise, J. Mass Spectrom. (1997), 32, 677-688). Ce type de source est particulièrement bien adapté, lorsque les peptides sont préalablement séparés par chromatographie liquide en phase inverse. Néanmoins, le rendement d'ionisation des peptides peut varier en fonction de la concentration et de la nature des différentes espèces en présence. Ce phénomène se traduit par un effet matrice bien connu de l'homme de l'art. Par ailleurs, il est également possible d'ioniser les peptides à partir d'un état solide grâce à une source MALDI (de l'anglais « Matrice Assisted Laser Desorbtion Ionisation »).

Ensuite, un analyseur quadripolaire (Q1) permet de filtrer les peptides protéotypiques en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure.

L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon.

Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être quantifiés.

Ce mode de fonctionnement présente une double sélectivité, en relation avec la sélection de l'ion précurseur d'une part et de la sélection de l'ion fragment de première génération d'autre part. La spectrométrie de masse en mode MRM est donc avantageuse pour la quantification.

L'intensité de courant induit par les ions fragments de première génération, mesurée dans le détecteur, est proportionnelle à la quantité d'ions fragments de première génération, elle-même proportionnelle à la quantité d'ions précurseurs, elle-même proportionnelle à la quantité de protéine à doser. La quantité de courant dosée, induit par les ions fragments de première génération, est donc directement proportionnelle à la quantité de la protéine à doser. Un calibrage est néanmoins nécessaire pour pouvoir corréler l'aire du pic mesurée, correspondant à la quantité de courant induit par les ions fragments de première génération, à la quantité d'ions fragments de première génération correspondant, et au final à la quantité de protéine à doser. Les couples (m/z)₁ et (m/z)₂, baptisés transitions, peuvent être dosés dans différents modèles de spectromètre de masse pouvant fonctionner en mode MRM ou en mode MS/MS (ou MS²). A titre d'exemple on peut citer les modèles de type triple quadripôle (L. Anderson & C. Hunter, MCP, 2006, 573-588...), ou de type trappe ionique (B. Han & R. Higgs, Brief Funct Genomic Proteomic. 2008 Sep;7(5):340-54), ou encore de type temps de vol (MALDI-TOF) (K.-Y. Wang et all, Anal Chem, 2008, 80(16) 6159-6167).

Les transitions utilisées pour doser une protéine doivent être caractéristiques de la protéine à doser et conduire aux dosages les plus sensibles, les plus spécifiques et les plus robustes possibles, en termes de reproductibilité et fiabilité. Ils doivent, pour cela, faire l'objet d'un choix minutieux.

Dans les méthodes développées pour la sélection des peptides protéotypiques (m/z)₁ et (m/z)₂, le choix est essentiellement basé sur l'intensité de la réponse. Pour plus de détails, on pourra se référer à V. Fusaro et al., Nature Biotech. 27 ; 2009 ; 190-198. La qualité de la séparation des peptides, en amont du dosage, la composition en acides aminés, l'absence d'un peptide précurseur identique dans une autre protéine susceptible d'être présente et l'état de charge du peptide précurseur sont également des facteurs qui sont pris en compte dans la sélection. En général, les masses générées par fragmentation de l'ion précurseur sont mesurées en mode MRM ou en mode MS/MS (MS²). Puis, l'ion fragment de première génération conduisant au signal le plus intense est sélectionné. Ensuite, les conditions de fragmentation de l'ion précurseur et les conditions d'analyse sont optimisées pour maximiser le signal obtenu. Il est connu qu'il est favorable de sélectionner des ions doublement chargés en Q1, et des ions fragments de première génération monochargés en Q3, de préférence de ratio m/z plus élevé que celui de l'ion précurseur dichargé.

Des logiciels commerciaux, tels que les logiciels MIDAS et MRM Pilote d'Applied Biosytems ou encore MRMaid (J. Mead et all, MCP, 15 nov 2008, E-pub) pourront être utilisés par l'homme de l'art, pour lui permettre de prédire tous les couples de transitions possibles. Il pourra également être fait appel à une base de données nommée PeptideAtlas, construite par F. Desiere et al., (Nucleic Acids Res. 2006, Jan 1 ; 34(database issue) : D655-8) pour compiler l'ensemble des transitions MRM de peptides, décrites par la communauté scientifique. Cette base PeptideAtlas est disponible en accès libre sur internet.

Une approche alternative pour sélectionner les peptides protéotypiques (m/z)₁ et (m/z)₂ consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunion utilisateurs (J. Mead et all, MCP, 15 nov 2008, E-pub). Elle permet de générer une liste de transitions candidates à partir des peptides identifiés expérimentalement par le logiciel SIEVE (Thermo Scientific).

Certains critères ont été détaillés par J. Mead et al. (MCP, supra) pour le choix des ions (m/z)₁ et (m/z)₂ et sont détaillés ci-après :
- Les peptides avec des sites de clivage interne, c'est à dire avec de la Lysine ou de l'Arginine interne, doivent être évités, sauf si la Lysine ou l'Arginine est suivie par de la Proline,
- Les peptides avec de l'Asparagine ou de la Glutamine doivent être évités car ils peuvent se désaminer,
- Les peptides avec de la Glutamine ou de l'Acide Glutamique en N-terminal doivent être évités car ils peuvent se cycliser spontanément,
- Les peptides avec de la Méthionine doivent être évités car ils peuvent être oxydés,
- Les peptides avec de la Cystéine doivent être évités car ils peuvent être modifiés de façon non reproductible lors d'une éventuelle étape de dénaturation, réduction et blocage des fonctions thiols,
- Les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- Les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- La sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

Par ailleurs, le plus souvent, pour assurer une sensibilité et une spécificité compatibles avec le dosage de protéine à une concentration de quelques ng/ml dans un fluide complexe (sang, sérum, plasma, urine, selle, crachat...), le dosage quantitatif par spectrométrie de masse doit être précédé, en plus de l'étape de digestion, d'autres étapes venant s'intercaler avec l'étape de digestion, comme par exemple :
Phase 1 : fractionnement des protéines pour éliminer les protéines majoritaires, ne correspondant pas à la protéine à doser, ou encore une purification de l'échantillon par toute technique appropriée : électrophorèse, chromatographie, immunocapture (Kulasingam et al., J. Proteome Res., 2008, 640-647). Néanmoins, cette dernière technique nécessite l'existence ou la préparation d'un anticorps spécifique dirigé contre la protéine à doser, ce qui peut être long et coûteux à obtenir. De plus, les performances ultérieures du dosage par spectrométrie de masse seront en partie liées à la qualité et à la spécificité de l'anticorps.
Phase 2 : dénaturation, réduction et blocage des fonctions thiols.
Phase 3 : digestion.
Phase 4 : fractionnement des peptides.

La phase 2 permet d'augmenter le rendement de digestion et assure une meilleure robustesse du dosage, en termes de reproductibilité et fiabilité. Les phases 1 et 4 sont optionnelles lorsqu'une grande sensibilité n'est pas requise (L. Anderson & C. Hunter, MCP, supra). En revanche, elles sont indispensables lorsqu'une grande sensibilité est nécessaire (quelques ng/ml). C'est ce qui a été démontré par T. Fortin et al., supra, H. Keshishian et al., MCP, 2007, 2212-2229 et V. Kulasingam et al., J. Proteome Res., 2008, 640-647, L. Anderson et al., J. Proteome Res., 2004, 235-2344 et US 2004/0072251. En effet, compte tenue de la précision des triples quadripôles en Q1 et Q3, de nombreux peptides - peptides isobariques ou quasiisobariques - peuvent générer des transitions identiques ou comprises dans les tolérances de masse de l'instrument (J. Sherman et al., Proteomics, 2008, 9 :1120-1123). Dans ce cas, l'injection simultanée d'un peptide protéotypique et d'un contaminant isobarique ou quasi-isobarique conduit à une quantification erronée par défaut de spécificité. La séparation des peptides par chromatographie en amont de la spectrométrie de masse apporte un niveau supplémentaire de spécificité en réduisant le nombre de peptides contaminants (M. Duncan et al., Proteomics, 2009, 9 :1124-1127). Mais cette étape de fractionnement supplémentaire peut ne pas être suffisante (H. Keshishian et al., supra), ce qui rend nécessaire de valider avec précaution toute transition utilisée pour quantifier une protéine. En outre, un procédé de dosage complet comprenant les phases 1, 2 et 4 en plus de l'étape de digestion, notamment lorsque des anticorps doivent être utilisés lors de la phase 1 ou 4, est long et coûteux.

Plus récemment une technique nommée MRM³ a été mise en oeuvre pour détecter des protéines. Cette technique consiste à sélectionner un ion fragment de première génération, et à le soumettre à une nouvelle fragmentation pour générer des ions fragments de deuxième génération. C'est alors ces ions fragments de deuxième génération qui sont détectés. J. Niessen et al. (MCP, 23 février 2009, E-Pub) notamment ont utilisé le triplé : précurseur / ion fragment de première génération / ion fragment de deuxième génération (baptisés transitions MRM³) **SEQ ID N°1** : VLLQTLR (2 fois chargé) / **SEQ ID N°2 :** LLQTLR (2 fois chargé) / **SEQ ID N°3 :** LQTLR (1 fois chargé). Toutefois, les auteurs ne commentent pas les raisons du choix de cette transition MRM³ en particulier, et se contentent uniquement de mettre en évidence la présence de la protéine cible, sans effectuer de dosage de cette dernière. Plus récemment, A. Izrael-Tomasevic et al. (Journal of Proteome Research, Targeting Interferon Alpha Subtypes in serum : A comparison of analytical approaches to the detection and quantitation of proteins in complex biological mixtures, date de publication sur intemet : 7 avril 2009) ont utilisé la MS³ pour la détection de IFN-alpha, la MS³ se distinguant de la MRM³ en ce que la totalité du spectre de MS³ est utilisé pour qualifier la nature du peptide protéotypique analysé. Pour cela, les auteurs sélectionnent, pour la détection de IFN-alpha 4, un ion moléculaire **SEQ ID N°4 :** HDFGFPQEEFGNQFQK (3 fois chargé) et l'ion y₁₅²⁺ **SEQ ID N°5 :** DFGFPQEEFGNQFQK (deux fois chargé) en ion fragment de première génération qui est soumis à une deuxième fragmentation, ainsi que pour l'ensemble des sous-types de IFN-alpha 4, un ion moléculaire SEQ **ID N°6 :** YSPCAWEVVR (deux fois chargé) et l'ion y₈²⁺ **SEQ ID N°7 :** PCAWEVVR (deux fois chargé) en ion fragment de première génération qui est soumis à une deuxième fragmentation. Aucun dosage quantitatif n'est effectué ni en mode MRM³, ni en mode MS³, et les auteurs soulignent que la quantification avec une trappe ionique de l'IFN-alpha n'est pas satisfaisante. Les auteurs constatent que même si le rapport signal/bruit des ions fragments de deuxième génération est amélioré en MS³, le nombre de coups est au moins mille fois plus faible qu'en FT-ICR et concluent qu'ils ont privilégié les approches SRM (MS2) et AMT (de l'anglais « Accurate Mass and Time ») (MS sans fragmentation).

Dans ce contexte, la présente invention se propose de fournir un nouveau procédé de dosage de protéine, mettant en oeuvre une technique de spectrométrie de masse qui soit fiable, facile à mettre en oeuvre, et ce à faible coût. L'invention se propose de fournir un procédé de dosage de protéine mettant en oeuvre la MRM³ et qui repose également sur un choix spécifique d'ions fragments de première génération, autorisant un tel dosage quantitatif satisfaisant.

L'invention concerne un procédé pour détecter, de manière quantitative, une protéine cible dans un échantillon comprenant les étapes suivantes :
a) le traitement de l'échantillon pour générer des peptides,
b) le dosage quantitatif, d'au moins un peptide protéotypique généré à partir de la protéine cible, par une technique de spectrométrie de masse dans laquelle :
   i) le peptide protéotypique est ionisé en ions précurseurs qui sont filtrés en fonction de leur masse m/z, et un ion précurseur de masse (m/z)₁ donné est sélectionné en fonction de la protéine cible recherchée,
   ii) l'ion précurseur sélectionné est fragmenté en ions fragments de première génération,
   iii) les ions fragments de première génération générés sont filtrés en fonction de leur masse m/z et un ion fragment de première génération de masse (m/z)₂ donné est sélectionné en fonction de la protéine cible recherchée,
   iv) l'ion fragment de première génération sélectionné est fragmenté en ions fragments de deuxième génération,
   v) au moins une partie des ions fragments de seconde génération sont détectés pour donner une série de mesures quantitatives,
   vi) au moins une mesure quantitative associée à un ion fragment de seconde génération est sélectionnée, et corrélée à la quantité du peptide protéotypique généré et à la quantité de protéine cible présente dans l'échantillon,
   caractérisé en ce que l'ion fragment de première génération de masse (m/z)₂ sélectionné est un peptide doublement chargé qui présente une proline et/ou une histidine en position 1.

La description qui suit permet de mieux comprendre l'invention. En préliminaire, certaines définitions des termes employés sont données ci-dessous.

Par « peptide », on entend un enchaînement d'au moins deux acides aminés. Les acides aminés en question peuvent être des acides aminés naturels, ou encore des acides aminés naturels modifiés tels que des acides aminés modifiés par action enzymatique.

En général, on nomme « peptide », un enchainement de 2 à 100 acides aminés. Un enchainement d'un nombre supérieur à 6 acides aminés pourra également être nommé « protéine », ces deux notions ne pouvant pas être séparées par une frontière claire. Dans le cadre de invention, on désignera par « protéine », les enchainements d'acides aminés initialement présents dans l'échantillon et par « peptides », les enchainements d'acides aminés issus du clivage d'au moins une liaison peptidique des protéines initiales ou de leurs peptides, par exemple par digestion, coupure chimique ou fragmentation dans un spectromètre de masse. Le terme « protéine » inclut les holoprotéines et les hétéroprotéines comme les nucléoprotéines, les lipoprotéines, les phosphoprotéines, les métalloprotéines et les glycoprotéines, les enzymes, les récepteurs, les anticorps, les antigènes.

Les protéines susceptibles d'être dosées par le procédé de l'invention sont, notamment, des protéines comportant au moins un peptide composé de n acides aminés, qui comporte lui-même au moins une proline au niveau des positions 2 à n-2 et/ou une histidine au niveau des positions 1 à n-2 qui sera obtenu après clivage de la protéine initiale. De préférence, ce peptide comprend de 1 à 15 acides aminés et au moins 6 acides aminés.

Par « peptide protéotypique », on entend un peptide généré par traitement d'une protéine pour la fragmenter en peptides, qui est caractéristique de ladite protéine ou d'une famille de protéines très proches, à laquelle appartient ladite protéine.

Par « échantillon », on entend tout échantillon susceptible de contenir la protéine à détecter. L'échantillon peut être d'origine biologique, soit animale, végétale ou humaine. Il peut alors correspondre à un prélèvement de fluide biologique (sang total, sérum, plasma, urine, liquide céphalorachidien, sécrétion organique, par exemple), un prélèvement tissulaire ou des cellules isolées. Ce prélèvement peut être utilisé tel quel ou, peut, à moins qu'il n'en soit spécifié autrement dans la description, subir préalablement à l'analyse, une préparation de type enrichissement, extraction, concentration, purification, selon des méthodes connues de l'homme du métier. L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produits lactés (yaourts, fromages,), de viande, de poisson, d'oeufs, de fruits, de légumes, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

Le ratio m/z correspond au rapport masse sur charge des peptides ionisés utilisés dans le cadre de l'invention. On parlera indifféremment, pour parler de ce ratio m/z, de rapport masse sur charge, de ratio, et même de masse. L'unité de ce ratio est en Th, mais on pourra également la donner en Da, par extension avec son appellation de « masse ».

La première étape a) du procédé selon l'invention correspond à un traitement des protéines contenues dans l'échantillon d'intérêt. L'ensemble des protéines de l'échantillon sont traitées pour fragmenter les protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré. Par rapport au traitement physico-chimique, il préserve d'avantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de Staphylococcus aureus qui clive la liaison peptidique au niveau du groupe carboxylique du résidu Glu ;
- les enzymes non-spécifiques comme la thermolysine provenant de la bactérie Bacillus thermoproteolyticus qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

Une telle étape de traitement permet de transformer les grosses molécules que sont les protéines présentes dans l'échantillon, en peptides, qui sont de plus petites molécules. La sensibilité de la détection obtenue ultérieurement par spectrométrie de masse se trouve ainsi augmentée. En outre, l'étape de traitement permet de générer plusieurs peptides protéotypiques, également nommés peptides rapporteurs pour une protéine cible donnée. La spécificité de chaque peptide protéotypique doit être vérifiée en s'assurant, par exemple, qu'aucune autre protéine ne comporte une séquence peptidique identique, ou qu'aucune autre transition n'interfère. Le traitement permet ainsi d'augmenter la possibilité d'obtenir un ou plusieurs peptides protéotypiques spécifiques de la protéine à doser. Chaque peptide protéotypique permet de quantifier la protéine par un dosage indépendant. La spécificité des dosages MRM dans des fluides complexes, tels que les échantillons sanguins, n'est pas assurée. Aussi, dans un tel cas, le dosage de plusieurs peptides protéotypiques pour chaque protéine permettra de vérifier que chaque dosage indépendant conduit bien à la même dose. L'obtention de dosages indépendants correctement corrélés entre eux permettra donc de s'assurer de la spécificité et de la robustesse, en termes de fiabilité et reproductibilité, de chaque dosage pris individuellement.

Ainsi, dans la suite du procédé, chaque ion précurseur de ratio (m/z)₁ donné, qui va être sélectionné pour l'analyse par spectrométrie de masse, sera issu d'un peptide protéotypique de la protéine cible à doser.

En fonction de la complexité de l'échantillon, l'étape de traitement pourra être précédée d'une ou plusieurs étapes optionnelles. Une toute première étape de fractionnement des protéines présentes dans l'échantillon d'intérêt pourra être mise en oeuvre pour réduire la complexité de l'échantillon, avant le traitement des protéines restantes. Par fractionnement, on entend, de façon classique, une épuration du nombre de protéines présentes : cela peut consister en la suppression d'une ou plusieurs protéines au sein de l'échantillon ou une sélection d'une ou plusieurs protéines, dont la protéine à doser. Une telle étape peut consister en la déplétion des protéines majoritaires présentes dans l'échantillon telle que l'albumine, l'IgG, l'IgA..., ces dites protéines ne correspondant pas à la protéine d'intérêt à doser. Une telle déplétion peut, par exemple, être effectuée par chromatographie d'affinité. Cette déplétion permet de réduire la complexité de l'échantillon en réduisant le nombre de protéines présentes. Une déplétion de l'albumine a été proposée par la demanderesse (T. Fortin et al., *supra).* La déplétion d'un panel plus important de protéines a également été utilisée par d'autres équipes (H. Keshishian et al., supra et V. Kulasingam et al., supra). Ces techniques pourront être mises en oeuvre dans le cadre de l'invention. Néanmoins, la chromatographie d'affinité présente l'inconvénient de mettre en oeuvre un milieu chromatographique coûteux lorsqu'il s'agit d'une résine d'immunoaffinité. De plus, si la spécificité de la capture est insuffisante, la protéine à doser peut être, elle aussi, en partie retenue par la résine d'affinité et perdue pour le dosage ultérieur, comme mis en évidence par T. Fortin et al., *supra.*

Une autre alternative pour fractionner les protéines consiste à immunopurifier la protéine d'intérêt, par exemple par chromatographie d'affinité. Ce procédé permet de réduire drastiquement la complexité de l'échantillon en obtenant une fraction comportant seulement la protéine à doser (et éventuellement quelques protéines contaminantes). Une telle approche est décrite par Kulasingam et al., *supra.* Néanmoins, de façon avantageuse, le procédé selon l'invention ne mettra pas en oeuvre une telle technique nécessitant de disposer d'anticorps de la protéine à doser. En effet, le procédé selon l'invention, de part sa spécificité vis-à-vis de la protéine à doser, rend superflu un fractionnement des protéines conduisant à un nombre très réduit de protéines restantes.

Une autre alternative pour fractionner les protéines consiste à purifier l'échantillon à doser par électrophorèse de type SDS-PAGE, puis à découper la bande correspondant à la masse moléculaire de la protéine à doser, comme notamment décrit par S.A. Gerber et al., *supra.* D'une manière générale, toutes les techniques de fractionnement de protéines du type électrophorèse, chromatographie..., bien connues de l'homme de l'art, peuvent être utilisées pour réduire la complexité de l'échantillon.

Une autre étape optionnelle de dénaturation, de réduction puis de blocage des fonctions thiols des protéines pourra être mise en oeuvre, notamment, avant l'étape de traitement entrainant le clivage des protéines, et après l'étape de fractionnement des protéines, lorsque celle-ci est présente. Cette étape, bien qu'optionnelle, permet d'augmenter le rendement de traitement par digestion et assure une meilleure robustesse du dosage ultérieur. Du fait de leur conformation tridimensionnelle, certaines protéines résistent naturellement à l'action protéolytique des protéases (V. Brun et all, MCP, 2007, 2139-2149). Dans un tel cas, une étape de dénaturation, de réduction puis de blocage des fonctions thiols des protéines facilite l'action des protéases et assure un rendement de traitement par digestion similaire d'un échantillon à l'autre. L'ensemble des protéines présentes dans l'échantillon d'intérêt peuvent être dénaturées et réduites, avec, par exemple, de l'urée ou de la guanidine et du dithiothreitol (DTT) ou du tris-(2-carboxyéthyl)phosphine (TCEP) (M. Sun et al., Bioconjug. Chem. 2005, 16(5) :1282-1290). Les fonctions thiols libres générées sont alors bloquées, avec, par exemple, de l'iodoacétamide ou de l'acrylamide (B. Herbert et al. Electrophoresis, 2001, 22 :2046-2057).

Selon le procédé de l'invention, au moins un peptide protéotypique généré lors de l'étape de traitement est dosé par spectrométrie de masse. En général, un seul peptide protéotypique par protéine, est dosé. Il est néanmoins possible de doser plusieurs peptides par protéine. Il est, également, possible de doser successivement des peptides protéotypiques de différentes protéines. L'ensemble des peptides générés lors de l'étape de traitement peuvent être injectés dans le spectromètre de masse. Mais le plus souvent, les peptides générés seront fractionnés, notamment pour focaliser le dosage sur des peptides protéotypiques de la protéine d'intérêt. Un tel fractionnement des peptides peut, par exemple, être réalisé par des techniques d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al., *supra,* H. Keshishian et al. *supra.* Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatrographie, pour le fractionnement des peptides, consiste à purifier spécifiquement les N-glycopeptides (J. Stal-Zeng et all, MCP, 2007, 1809-1817 et demande de brevet WO 2008/066629). Néanmoins, une telle purification ne permet que la quantification des peptides ayant subi une modification post-traductionnelle de type N-glycosylation. Or toutes les protéines ne sont pas glycosilées, ce qui limite donc son utilisation.

Une autre alternative pour fractionner les peptides consiste à immunopurifier le peptide d'intérêt, par exemple par chromatographie d'affinité. Ce procédé permet de réduire drastiquement la complexité de l'échantillon en obtenant une fraction comportant seulement le peptide à doser (et éventuellement quelques peptides contaminants). Une telle approche nommée SISCAPA est décrite dans L. Anderson et al., *supra,* et dans la demande de brevet US 2004/0072251. Néanmoins, l'obtention d'anticorps anti-peptide spécifiques peut être difficile et dans ce cas, le procédé selon l'invention ne mettra pas en oeuvre une telle technique. De plus, le procédé selon l'invention, de part sa spécificité vis-à-vis de la protéine à doser, rend superflu un fractionnement par immunoaffinité des peptides conduisant au seul peptide protéotypique à doser.

En conclusion, dans le cadre du procédé selon l'invention, le dosage quantitatif des peptides générés par spectrométrie de masse (correspondant à l'étape b)) est, de préférence, précédé d'une séparation par chromatographie ou électrophorèse des peptides générés à l'étape a). Une telle séparation par chromatographie utilisera, de préférence, une séparation par chromatographie en phase inverse, une chromatographie avec micro-débit, c'est-à-dire un débit de 100µl à 500 µl par minutes, ou encore une purification par extraction en phase solide (nommé en anglais Solide Phase Extraction ou SPE).

L'étape b) du procédé selon l'invention consiste en la détection d'au moins un peptide protéotypique généré, lors des étapes précédentes, par une technique de spectrométrie de masse. En général, un seul peptide protéotypique est dosé par protéine, mais il pourrait être possible d'en doser deux ou plus, leur ordre d'arrivée étant conditionné en amont du spectromètre de masse, par la technique de fractionnement des peptides sélectionnés.

Différents modèles de spectromètre de masse sont utilisables, dans le cadre de l'invention. Ces modèles doivent permettre trois étapes de séparation en fonction du ratio m/z avec deux fragmentations successives intercalées entre les étapes de séparation, c'est-à-dire un type d'analyse appelée MS/MS/MS ou encore MS³. A titre d'exemple, on peut citer les modèles de type triple quadripôle (L. Anderson & C. Hunter, MCP, 2006, 573-588...), ou de type trappe ionique (B. Han & R. Higgs, Brief Funct Genomic Proteomic. 2008 Sep;7(5):340-54), ou encore de type temps de vol (MALDI-TOF) (K.-Y. Wang et all, Anal Chem, 2008, 80(16) 6159-6167). Les modèles de type triple quadripôle hybride, intégrant une trappe ionique, sont néanmoins préférés. Les peptides à doser, le plus souvent en solution, sont injectés et dosés dans le spectromètre de masse choisi.

Dans une première étape i), les peptides issus du traitement des protéines entrainant leur clivage, qui sont injectés dans le spectromètre de masse, sont ionisés en ions moléculaires, nommés précurseurs étant donné qu'ils vont être soumis ultérieurement à fragmentation pour générer des ions fragments. Pour être injectés, dans le spectromètre de masse, les peptides sont en général dissous dans une solution aqueuse. L'échantillon à doser sera donc, de préférence, liquide. La source d'ions dans laquelle les molécules sont ionisées est, par exemple, du type MALDI *(Matrix Assisted Laser Desorption Ionisation*) ou Electrospray (ESI de l'anglais *« ElectroSpray Ionisation* »). Une ionisation par électrospray, réalisée dans des conditions douces, notamment à pression atmosphérique et température ambiante, est néanmoins préférée.

Les ions précurseurs générés sont alors filtrés en fonction de leur masse m/z et un ion précurseur de masse (m/z)₁ donnée est sélectionné en fonction de la protéine cible recherchée. Pour cela, classiquement, les ions sont accélérés dans un champ électrique, puis dirigés dans un champ électrique et/ou magnétique selon une trajectoire qui dépend de leur rapport masse/charge (m/z). Une variation du champ électrique et/ou magnétique appliqué, permet de faire varier la trajectoire des ions précurseurs, et permet ainsi de sélectionner le rapport (m/z)₁ souhaité. Selon une caractéristique préférée de l'invention, l'ion précurseur sélectionné est un peptide doublement chargé qui contient un nombre n de 6 à 15 acides aminés, et qui présente au moins une proline au niveau des positions 2 à n-2 et/ou une histidine au niveau des positions 1 à n-2. Une telle sélection va permettre d'obtenir une bonne spécificité et sélectivité dans les étapes suivantes avec une amélioration du rapport signal sur bruit. Selon un mode de réalisation préféré, l'ion précurseur sélectionné est doublement chargé et présente au moins deux prolines ou une proline et une histidine. En effet, l'utilisation d'ions précurseurs ayant deux prolines ou une proline et une histidine génère encore moins de fragmentations secondaires en MS³ et est encore plus adaptée pour obtenir le dosage quantitatif le plus performant.

Par peptide comprenant une proline (ou une histidine) au niveau des positions 2 à n-2, on entend que cette dernière peut être en position 2, 3, 4, 5 ... n-5, n-4, n-3 ou n-2. La position 1 correspond à la partie N-terminale et la position n à la partie C-terminale, n correspondant au nombre d'acides aminés présents dans le peptide.

L'ion précurseur sélectionné, en fonction de la protéine cible recherchée, est ensuite fragmenté en ions fragments de première génération dans une étape ii). Les ions fragments de première génération générés sont, ensuite, filtrés en fonction de leur masse m/z et un ion fragment de première génération de masse (m/z)₂ donné est sélectionné, dans une étape iii). Selon une caractéristique essentielle de l'invention, l'ion fragment de première génération sélectionné correspond à un peptide dichargé comportant une proline ou une histidine en position 1 (N-terminale). En effet, lorsque l'ion précurseur comporte une proline ou une histidine, il existe un ion fragment de première génération qui correspond à une fragmentation de la liaison peptidique en N-terminal de la proline ou de l'histidine, respectivement. Dans l'étape iv) du procédé selon l'invention, l'ion fragment de première génération sélectionné va subir une nouvelle fragmentation et le choix particulier de l'ion précurseur et de l'ion fragment de première génération conduit à une bonne fragmentation et à l'obtention de pics intenses. Dans le cadre de l'invention, les ions précurseurs de ratio (m/z)₁ et les ions fragments de première génération de ratio (m/z)₂, sont choisis, de manière à être caractéristiques de la protéine à doser et conduire aux dosages les plus sensibles, les plus spécifiques et les plus robustes possibles, en termes de reproductibilité et fiabilité.

Les deux fragmentations successives seront classiquement réalisées par collision avec un gaz inerte, comme de l'azote ou de l'argon au sein d'un champ électrique, ou encore par la seule application d'une différence de potentiel, par exemple dans un tube de temps de vol. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e⁻V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Les étapes i) à iii) correspondent aux étapes utilisées classiquement dans un dosage MRM. De telles étapes peuvent être menées dans un triple quadripôle notamment. Un triple quadripôle comporte deux analyseurs quadripolaires (nommés Q1 et Q3) en série, séparés par une cellule de collision (nommée q2) souvent constituée d'un quadripôle plus court. Bien évidemment, il est nécessaire de mettre en oeuvre le procédé selon l'invention, dans un spectromètre de masse ayant la capacité de fragmenter les ions fragments de première génération en ions fragments de deuxième génération. La fragmentation d'un ion fragment de première génération en ion fragment de deuxième génération et la sélection de l'ion fragment de première génération, dans le cadre de l'invention, peut par exemple être réalisée en Q3 d'un triple quadripôle comportant une trappe ionique. Des triples quadripôles hybrides possédant une capacité de trappe ionique dans le quadripôle Q3 pourront être utilisés. De tels instruments sont commercialisés par la société Applied Biosytems sous les appellations de 3200QTRAP®, de 4000QTRAP® ou de AB SCIEX QTRAP® 5500. Il est également possible de fragmenter un ion fragment de première génération en mode MS³ dans tout analyseur de type trappe ionique. Dans ce cas, l'ion précurseur est sélectionné dans la trappe ionique, puis fragmenté en ions fragments de première génération. Un ion fragment de première génération est à son tour sélectionné puis fragmenté en ions de seconde génération dans le même dispositif de type trappe ionique. Les ions de seconde génération sont finalement détectés en les expulsant séquentiellement hors de la trappe ionique vers le détecteur.

Dans le cadre de l'invention, classiquement, l'analyseur quadripolaire (Q1) permet de filtrer les ions moléculaires précurseurs issus du peptide protéotypique sélectionné, en fonction de leur ratio masse sur charge (m/z)₁. Seuls les peptides ayant le ratio masse/charge de l'ion précurseur recherché (m/z)₁ sont transmis dans le deuxième quadripôle (q2). L'analyseur q2 permet de fragmenter les ions précurseurs de ratio masse/charge (m/z)₁ en ion fragment de première génération. La fragmentation est généralement obtenue par collision des peptides précurseurs avec un gaz inerte, comme de l'azote ou de l'argon. Les ions fragments de première génération sont, alors, transmis dans un troisième quadripôle (Q3) qui filtre les ions fragments de première génération en fonction d'un ratio masse sur charge (m/z). Seul un ion fragment de première génération ayant le ratio masse/charge (m/z)₂ d'un fragment caractéristique du peptide protéotypique recherché va subir les dernières étapes du procédé selon l'invention. En effet, le procédé selon l'invention comporte une fragmentation supplémentaire : dans une étape ultérieure iv), l'ion fragment de première génération sélectionné de masse (m/z)₂ est fragmenté en ions fragments de deuxième génération. Après la sélection en Q3 d'un ion fragment de première génération ayant le ratio masse/charge (m/z)₂ d'un fragment du peptide protéotypique recherché, cet ion fragment de première génération est à son tour fragmenté, par exemple grâce à une trappe ionique. Différents ions fragments de deuxième génération issus de la fragmentation de l'ion fragment de première génération sont alors obtenus. Ces différents ions de seconde génération vont alors être expulsés vers un détecteur, par l'analyseur de masse constitué par exemple d'une trappe ionique ou d'un quadripôle. L'expulsion est, par exemple, réalisée séquentiellement hors de la trappe ionique, par l'application d'une tension modulée en fonction du temps par une radio fréquence.

Afin de détecter le courant induit, les ions fragments de deuxième génération sont dirigés vers un détecteur qui collecte les ions fragments de deuxième génération qui arrivent à des temps différents en fonction du rapport m/z et qui amplifie le signal associé aux ions. Selon une variante de mise en oeuvre du procédé selon l'invention, à l'étape v), l'intensité du courant induit par les ions fragments de deuxième génération est détectée en fonction du temps et le signal obtenu sur une période donnée est décomposé en un spectre de masse des différents ions présents en fonction de leur masse m/z, de manière à obtenir un pic de masse associé à chacun des ions de seconde génération détectés présents sur la période donnée, et le signal correspondant au courant d'au moins un ion de deuxième génération sélectionné est recomposé, et l'intensité du courant correspondant mesurée est la mesure quantitative sélectionné à l'étape vi). En général, le signal total est obtenu par fraction de temps, ou période t. La durée des périodes t est fonction de la vitesse de balayage de l'analyseur de masse et de la plage de masses à balayer, elle est généralement inférieure à une seconde. Le signal total obtenu sur chaque période t donnée est décomposé en un spectre de masse des différents ions présents en fonction de leur masse m/z, de manière à obtenir un pic de masse associé à chacun des ions de seconde génération détectés sur cette période t. Les ions de seconde génération sont observés pendant plusieurs périodes t consécutives, correspondant à la période T durant laquelle le peptide protéotypique est élué. La période t au cours de laquelle le signal des ions fragments de deuxième génération induits par le peptide protéotypique, est maximal correspond au temps d'élution, ou temps de rétention, du peptide protéotypique. Le temps d'élution du peptide protéotypique peut être déterminé, par exemple, de façon classique par MRM, à partir d'une solution du peptide protéotypique purifié ou d'une solution de la protéine cible purifiée ou d'une solution dans laquelle l'un ou l'autre est particulièrement abondant. La durée minimale de la période T est classiquement de 5 à 30s. Cependant, la durée de la période T est généralement allongée, pour que la mesure du signal ne soit pas affectée par une micro-variation du temps d'élution du peptide protéotypique. La durée de la période T peut ainsi être allongée jusqu'à atteindre la totalité du temps de la séparation chromatographique. Cependant, de façon classique, la période T est généralement fixée entre une minute et cinq minutes, centrée autour du temps d'élution du peptide protéotypique. Le signal correspondant au courant d'au moins un ion de deuxième génération, spécifique du peptide protéotypique issu de la protéine à quantifier, peut être sélectionné pour chaque fraction de temps t, durant la période T. Ce signal peut alors être extrait du signal total. L'intensité du courant correspondant à la somme des intensités mesurées à chaque instant t de la période T ou d'une fraction de la période T correspondra à la mesure quantitative sélectionnée à l'étape vi). En effet, comme cela est détaillé à l'exemple 1, le signal correspondant au courant généré par les ions détectés, en fonction du temps, peut être décomposé pour chaque période t donnée (qui est en fait une très courte période), et sommé, par exemple, pour la fraction la plus représentative de la période T, pour obtenir un spectre de masse des différents ions présents en fonction de leur masse m/z. On obtient ainsi un pic de masse associé à chacun des ions de seconde génération détectés à l'étape v) et présents sur la fraction la plus représentative de la période T. Il est ensuite possible de sélectionner un ou plusieurs ions de seconde génération de masse donnée et de recomposer le signal correspondant au courant des ions sélectionnés. On obtient, ainsi, la somme des intensités du courant induit par les ions fragments de chaque période t, survenant durant la fraction la plus représentative de la période T, par intégration de la somme des signaux observés durant les périodes t consécutives de la fraction la plus représentative de la période T correspondant à l'élution du peptide protéotypique. Cette somme correspond, par exemple, à la mesure quantitative, qui va permettre de déterminer la quantité de peptide protéotypique présent. La première et la dernière période t de la fraction la plus représentative de la période T dont les signaux sont sommés pourront correspondre à la première et la dernière période t pour lesquelles le signal est supérieur au bruit de fond du détecteur. L'intensité de courant ainsi mesurée pourra servir de mesure quantitative permettant de déterminer la quantité de peptide protéotypique présent, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative. De préférence, à l'étape vi), on sélectionne la mesure quantitative associée à l'ion fragment de deuxième génération, présentant le pic m/z le plus intense. Il est également possible de sommer les mesures quantitatives de différents ions fragments de deuxième génération. Dans ce cas, à l'étape vi), la corrélation est, par exemple, effectuée à partir de la somme d'au moins deux mesures quantitatives (notamment deux ou trois), chacune étant associée aux ions fragments de deuxième génération présentant les pics m/z les plus intenses.

Un ensemble informatique de traitement des données permet de transformer les informations reçues par le détecteur en spectre de masse. L'intensité de courant induit par les ions fragments de deuxième génération sélectionnés, mesurée dans le détecteur, est proportionnelle à la quantité d'ions fragments de deuxième génération, elle-même proportionnelle à la quantité d'ions fragments de première génération, elle-même proportionnelle à la quantité d'ions précurseurs obtenus par ionisation du peptide protéotypique sélectionné, elle-même proportionnelle à la quantité de protéine à doser. La quantité de courant dosé, induit par les ions fragments de deuxième génération, est donc directement proportionnelle à la quantité de la protéine à doser. La sélection d'au moins une mesure quantitative associée à un ion de seconde génération, et la corrélation de cette mesure quantitative à la quantité de peptide protéotypique généré et à la quantité de protéine présente dans l'échantillon, permet d'obtenir un dosage quantitatif.

Un calibrage est nécessaire pour pouvoir corréler l'aire du pic mesurée, correspondant à l'intensité de courant induit par le ou les ions fragments de deuxième génération, à la quantité d'ions fragments de deuxième génération correspondants, qui pourra elle-même être corrélée à la quantité d'ions fragments de première génération correspondante, qui pourra elle-même être corrélée à la quantité d'ions précurseurs, qui pourra elle-même être corrélée à la quantité de la protéine d'intérêt. Pour cela, les calibrages classiquement utilisés dans les dosages MRM pourront être mis en oeuvre, dans le cadre de l'invention. Les dosages MRM sont classiquement calibrés à l'aide de standards externes ou, de préférence, à l'aide de standards internes tels que décrits par T. Fortin et al., *supra.* La corrélation entre la mesure quantitative et la quantité de peptide protéotypique, et par la suite de protéine cible, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N)

L'utilisation de peptides lourds, comme standards internes (AQUA), a également été proposée par S.A. Gerber et al., *supra* et dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. (Nat. Protoc. 2006, 1:1029-1043) sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. (MCP, 2007, 2139-2149) ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. *supra* ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage, dans le cadre de l'invention.

Le procédé selon l'invention pourra donc être mis en oeuvre pour le dosage quantitatif et qualitatif de protéine, pour des applications de diagnostic *in vitro,* notamment. Le **TABLEAU 1A** ci-dessous détaille un certain nombre de protéines pouvant être dosées par le procédé selon l'invention, ainsi que le peptide protéotypique sélectionné. **TABLEAU 1A**

| Protéine | Masse m/z de l'ion précurseur M2H+ | Masse du peptide protéotypique | Position du peptide protéotypique au sein de la protéine | Séquence du peptide protéotypique |
|---|---|---|---|---|
| Plastine-1 | 730,37935 | 1458,7587 | 349-362 | **SEQ ID N°8 :** QFVTPADVVSGNPK |
| Plastine-1 | 538,29145 | 1074,5829 | 42-51 | **SEQ ID N°9 :** EASLPLPGYK |
| Plastine-1 | 535,81255 | 1069,6251 | 266-274 | **SEQ ID N°10 :** LSPEELLLR |
| Ezrin | 553,2918 | 1104,5836 | 237-245 | **SEQ ID N°11 :** IGFPWSEIR |
| Aminoacylase 1 | 446,2309 | 890,4618 | 277-284 | **SEQ ID N°12 :** VAPDVDFK |
| Fatty acid-binding protein (L-FABP) | 606,35205 | 1210,7041 | 21-31 | **SEQ IDN°13 :** AIGLPEELIQK |
| Protéine Disulfide-isomérase | 983,522 | 1965,044 | 231-247 | **SEQ ID N°14 :** HNQLPLVIEFTEQTAPK |
| Protéine Disulfide-isomerase | 761,87715 | 1521,7543 | 18-30 | **SEQ ID N°15 :** DAPEEEDHVLVLR |
| Protéine Disulfide-isomerase | 726,8506 | 1451,7012 | 327-338 | **SEQ ID N°16 :** YKPESEELTAER |
| Protéine Disulfide-isomerase | 541,8384 | 1081,6768 | 255-263 | **SEQ ID N°17** : THILLFLPK |
| Protéine Disulfide-isomerase | 486,2678 | 970,5356 | 402-409 | **SEQ ID N°18 :** QLAPIWDK |
| Protéine Disulfide-isomerase | 465,2625 | 928,525 | 437-444 | **SEQ ID N°19 :** VHSFPTLK |
| Protéine Disulfide-isomerase | 456,22085 | 910,4417 | 445-452 | **SEQ** ID **N°20 :** FFPASADR |
| Protéine Disulfide-isomerase | 432,23345 | 862,4669 | 58-65 | **SEQ ID N°21 :** ALAPEYAK |
| Protéine Disulfide-isomerase | 431,1965 | 803,3716 | 310-316 | **SEQ ID N°22 :** EECPAVR |
| Keratin, type 1 cytoskeletal 20 | 932,482 | 1846,9691 | nov-28 | **SEQ ID N°23 :** SLSSSLQAPVVSTVGMQR |
| Keratin, type 1 cytoskeletal 20 | 658,8188 | 1315,6376 | 308-318 | **SEQ ID N°24 :** ESLEHTLEETK |
| Keratin, type 1 cytoskeletal 20 | 647,32965 | 1292,6593 | 29-42 | **SEQ ID N°25 :** LGTTPSVYGGAGGR |
| Keratin, type 1 cytoskeletal 20 | 583,2716 | 1164,5432 | 104-112 | **SEQ ID N°26 :** QWYETNAPR |
| Keratin, type 1 cytoskeletal 20 | 544,7891 | 1087,5782 | 179-187 | **SEQ ID N°27 :** VFDDLTLHK |
| 14-3-3 protéine sigma | 528,2675 | 1054,535 | 161-169 | **SEQ ID N°28 :** EMPPTNPIR |
| Protéine S100-A11 Calqizarin | 510,7502 | 1019,5004 | 04-déc | **SEQ ID N°29 :** ISSPTETER |
| Protéine S100-A11 Calgizarin | 386,69975 | 771,3995 | 56-62 | **SEQ ID N°30 :** DPGVLDR |

Les protéines étudiées correspondent au numéro suivant dans la base Swiss Prot : Plastine-1 (Q14651), Ezrin (P15311), Aminoacylase 1(Q03154), Protéine Disulfide-isomerase (P07237), Keratin, type 1 cytoskeletal 20 (P35900), 14-3-3 protéine sigma (P31947), Protéine S100-A11 Calgizarin (P31949).

La masse théorique du peptide protéotypique est déterminée à partir de sa séquence en acides aminés. De nombreux logiciels permettent ce calcul, tels que MRM Pilot (Applied Biosystems), Sequence Editor (Bruker Daltonik, Brême, Allemagne)... Le calcul donné **TABLEAU 1A** a été réalisé avec MRM Pilot. La masse théorique de l'ion précurseur 2 fois chargés (M2H⁺) est déterminée en ajoutant la masse de deux protons à la masse théorique du peptide protéotypique, et en divisant la somme obtenue par 2.

Le **TABLEAU 1B** donne, pour chaque protéine, en fonction de l'ion précurseur doublement chargé sélectionné, la position des prolines et histidines présentes et le nombre d'acides aminés présents (AA signifiant acide aminé).

**TABLEAU 1B**

| Protéine | Séquence du peptide protéotypique | Position 1ère Proline | Position 2ème Proline | Nbre AA entre 1ère Proline et C-term | Position 1ère His | Nbre AA entre 1ère His et C-term | Nbre AA total |
|---|---|---|---|---|---|---|---|
| Plastine-1 | **SEQ ID N°8** | 5 | 13 | 9 | - | - | 14 |
| Plastine-1 | **SEQ ID N°9** | 5 | 7 | 5 | - | - | 10 |
| Plastine-1 | **SEQ ID N°10** | 3 | - | 6 | - | - | 9 |
| Ezrin | **I SEQ ID N°11** | 4 | - | 5 | - | - | 9 |
| Aminoacylase 1 | **SEQ ID N°12** | 3 | - | 5 | - | - | 8 |
| Fatty acid-binding protéine (L-FABP) | **SEQ ID N°13** | 5 | - | 6 | - | - | 11 |
| Protéine Disulfide-Isomerase | **SEQ ID N°14** | 5 | 16 | 12 | 1 | 16 | 17 |
| Protéine Disulfide-Isomerase PDI A1 | **SEQ ID N°15** | 3 | - | 10 | 8 | 5 | 13 |
| Protéine Disulfide-Isomerase | **SEQ ID N°16** | 3 | - | 9 | - | - | 12 |
| Protéine Disulfide-Isomerase | **SEQ ID N°17** | 8 | - | 1 | 2 | 7 | 9 |
| Protéine Disulfide-Isomerase | **SEQ ID N°18** | 4 | - | 4 | - | - | 8 |
| Protéine Disulfide-Isomerase | **SEQ ID N°19** | 5 | - | 3 | 2 | 6 | 8 |
| Protéine Disulfide-Isomerase | **SEQ ID N°20** | 3 | - | 5 | - | - | 8 |
| Protéine Disulfide-Isomerase | **SEQ ID N°21** | 4 | - | 4 | - | - | 8 |
| Protéine Disulfide-Isomerase | **SEQ ID N°22** | 4 | - | 3 | - | - | 7 |
| Keratin, type 1 cytoskeletal 20 | **SEQ ID N°23** | 9 | - | 9 | - | | 18 |
| Keratin, type 1 cytoskeletal 20 | **SEQ ID N°24** | - | - | | 5 | 6 | 11 |
| Keratin, type 1 cytoskeletal 20 | **SEQ ID N°25** | 5 | - | 9 | - | | 14 |
| Keratin, type 1 cytoskeletal 20 | **SEQ ID N°26** | 8 | - | 1 | - | | 9 |
| Keratin, type 1 cytoskeletal 20 | **SEQ ID N°27** | - | - | | 8 | 1 | 9 |
| 14,3,3 protein sigma | **SEQ ID N°28** | 3 | 4 | 6 | - | | 9 |
| Protéine S100-A11 Calgizarin | **SEQ ID N°29** | 4 | - | 5 | - | | 9 |
| Protéine S100-A11 Calgizarin | **SEQ ID N°30** | 2 | - | 5 | - | | 7 |

Seul le peptide protéotypique HNQLPLVIEFTEQTAPK **(SEQ ID N°14)** comportant 17 acides aminés, dans le cas de la protéine Protéine Disulfide-Isomerase et le peptide SLSSSLQAPVVSTVGMQR **(SEQ ID N°23)** comportant 18 acides aminés, dans le cas de la protéine Keratin, type 1 cytoskeletal 20, ne conduisent à aucune détection de l'ion précurseur doublement chargé correspondant. Le **TABLEAU 1C** donne, pour chaque protéine, l'ion fragment de 1ère génération doublement chargé sélectionné, avec la position des prolines et histidines présentes.

**TABLEAU 1C**

| | Séquence du peptide protéotypique | Fragment de première génération doublement chargé | Position de la première Proline | Position de la première Histidine |
|---|---|---|---|---|
| Plastine-1 | **SEQ ID N°8** | **SEQ ID N°31 :** PADVVSGNPK | 1 | - |
| Plastine-1 | **SEQ ID N°9** | **SEQ ID N°32 :** PLPGYK | 1 | - |
| Plastine-1 | **SEQ ID N°10** | **SEQ ID N°33 :** PEELLLR | 1 | - |
| Ezrin | **SEQ ID N°11** | **SEQ ID N°34 :** PWSEIR | 1 | - |
| Aminoacylase 1 | **SEQ ID N°12** | **SEQ ID N°35 :** PDVDFK | 1 | - |
| Fatty acid-binding protéine (L-FABP) | **SEQ ID N°13** | **SEQ ID N°36 :** PEELIQK | 1 | - |
| Protéine Disulfide-isomerase | **SEQ ID N°14** | - | - | - |
| Protéine Disulfide-isomerase | **SEQ ID N°15** | **SEQ ID N°37** : PEEEDHVLVLR | 1 | - 6 |
| Protéine Disulfide-isomerase | **SEQ ID N°16** | **SEQ ID N°38 :** PESEELTAER | 1 | - |
| Protéine Disulfide-isomerase | **SEQ ID N°17** | **SEQ ID N°39 :** HILLFLPK | 7 | 1 |
| Protéine Disulfide-isomerase | **SEQ ID N°18** | **SEQ ID N°40 :** PIWDK | 1 | - |
| Protéine Disulfide-isomerase | **SEQ ID N°19** | **SEQ ID N°41 :** HSFPTLK | 4 | 1 |
| Protéine Disulfide-isomerase | **SEQ ID N°20** | **SEQ ID N°42 :** PASADR | 1 | - |
| Protéine Disulfide-isomerase | **SEQ ID N°21** | **SEQ ID N°43** : PEYAK | 1 | - |
| Protéine Disulfide-isomerase | **SEQ ID N°22** | **SEQ ID N°44 :** PAVR | 1 | - |
| Keratin, type 1 cytoskeletal 20 | **SEQ ID N°23** | - | - | - |
| Keratin, type 1 cytoskeletal 20 | **SEQ ID N°24** | **SEQ ID N°45 :** HTLEETK | - | 1 |
| Keratin, type 1 cytoskeletal 20 | **SEQ ID N°25** | **SEQ ID N°46** : PSVYGGAGGR | 1 | - |
| Keratin, type 1 cytoskeletal 20 | **SEQ ID N°26** | - | - | - |
| Keratin, type 1 cytoskeletal 20 | **SEQ ID N°27** | - | - | - |
| 14-3-3 protéine sigma | **SEQ ID N°28** | **SEQ ID N°47** : PPTNPIR | 1 | - |
| Protéine S100-A11 Calgizarin | **SEQ ID N°29** | **SEQ ID N°48** : PTETER | 1 | - |
| Protéine S100-A11 Calgizarin | **SEQ ID N°30** | **SEQ ID N°49** : PGVLDR | 1 | - |

Les exemples ci-après, en référence aux Figures annexées, n'ont aucun caractère limitatif et permettent d'illustrer l'invention.
La **Figure 1A** présente un exemple de signal obtenu, en fonction du temps sous forme d'un chromatogramme d'ions total.
La **Figure 1B** représente le spectre de masse des ions fragments de seconde génération élués entre 11,70 et 11,90 minutes sur la **Figure 1A****.**
La **Figure 1C** montre la somme des chromatogrammes d'ions extraits correspondant aux masses des fragments de seconde génération y5, y6, y7-H₂O, y7 et y8 du peptide protéotypique LSEPAELTDAVK **(SEQ ID N°50)** du PSA, lissé sur trois points, de la **Figure 1A****.**
La **Figure 1D** est un exemple de courbe de calibration.
La **Figure 2A** représente le chromatogramme obtenu pour le peptide LSEPAELTDAVK **(SEQ ID N°50)** natif (temps de rétention 11,78 min) et la **Figure 2B** celui du peptide LSEPAELTDAVK **(SEQ ID N°50)** lourd (temps de rétention 11,79 min).
Les **Figures 3****,** **4A** et **4B** sont d'autres exemples de courbe de calibration.
Les **Figures 5A****, SB** et **5C** sont différents spectres de masse obtenus avec différentes énergies de collision, respectivement 40, 35 et 30 e⁻V.
La **Figure 5D** représente l'évolution de l'intensité du courant induit, en fonction de l'énergie de collision (CE) utilisée pour la formation des ions fragments de première génération.
Les **Figures 6A, 6B** et **6C** représentent les spectres de masse des ions fragments de seconde génération obtenus du peptide protéotypique SAPSPLTYR **(SEQ ID N°53)** de la protéine Tp435 en choisissant, comme ion fragment de première génération, soit l'ion fragment monochargé le plus intense **(****Figure 6A** **-** élution entre 0,098 et 0,300 min), soit l'ion fragment contenant une proline sous forme monochargé **(****Figure 6B** **-** élution entre 0,101 et 0,306 min), soit ce même ion sous forme dichargé **(****Figure 6C**-élution entre 0,102 et 0,298 min).

### Exemple 1 :Principe de dosage quantitatif par MRM³ avec calibration externe d'un marqueur tumoral : l'antigène spécifique de la prostate (PSA)

Le PSA (selon l'acronyme anglais pour Prostate Specific Antigen, fourni par la société anglaise Scipac) est implémenté aux concentrations décrites ci-dessous dans du sérum de femme (Etablissement Français du Sang) pour constituer les points de la gamme d'étalonnage :
- 35 µl de PSA à 1,14mg/ml dans 165µl de sérum de femme pour obtenir un point à 200µg/ml
- 50 µl du point à 200µg/ml dans 150µl de sérum de femme pour obtenir un point à 50µg/ml
- 40 µl du point à 50µg/ml dans 160µl de sérum de femme pour obtenir un point à 10µg/ml
- 20µl du point à 10µg/ml dans 180µl d'H₂O pour avoir un point à 1µg/ml
- 20µl du point à 1µg/ml dans 180µl d'H₂O pour avoir un point à 100ng/ml
- 20µl du point à 100ng/ml dans 180µl d'H₂O pour avoir un point à 10ng/ml
- 20µl du point à 10ng/ml dans 180µl d'H₂O pour avoir un point à 1ng/ml
- 20µl du point à 50µg/ml dans 180µl d'H₂O pour avoir un point à 5µg/ml
- 20µl du point à 5µg/ml dans 180µl d'H₂O pour avoir un point à 500ng/ml
- 20µl du point à 500ng/ml dans 180µl d'H₂O pour avoir un point à 50ng/ml 200µl de sérum de femme sont utilisés pour obtenir un point à 0ng/ml.

Les points de gamme et les échantillons sériques à doser, échantillons prélevés chez des patients atteints d'un cancer de la prostate ou d'une hyperplasie bénigne de la prostate, sont ensuite digérés selon le protocole suivant :
- Dilution d'un volume de 100µl de sérum dans 3ml de bicarbonate 50mM, pH=8,0
- Ajout du dithiothréitol (DTT) pour obtenir une concentration finale de 15mM
- Réduction à 60°C pendant 40minutes
- Refroidissement des tubes à température ambiante
- Ajout d'iodoacétamide pour obtenir une concentration finale de 25 mM
- Alkylation pendant 40minutes à température ambiante et à l'obscurité
- Ajout de trypsine avec un ratio de 1/30
- Digestion à 37°C pendant 4 heures
- Ajout du DTT pour obtenir une concentration finale de 10mM
- Réduction à 60°C pendant 40 minutes
- Refroidissement des tubes à température ambiante
- Ajout d'iodoacétamide pour obtenir une concentration finale de 15mM et permettre une alkylation des fonctions thiol à température ambiante et à l'obscurité pendant 40 minutes
- Ajout de trypsine avec un ratio massique de 1/30
- Digestion à 37°C pendant toute la nuit

Le sérum est ensuite dessalé et concentré selon le protocole suivant :
Acidification des échantillons avec de l'acide formique (soit 0,1% final)
Equilibrage des colonnes HLB Oasis Waters avec 1ml de méthanol puis 1 ml H₂O/acide formique 0,1%
Dépôt de l'échantillon qui s'écoule par gravité
Lavage avec 1 ml H₂O/acide formique 0,1%
Elution avec 1ml de méthanol 80% dans un mélange H₂O/acide formique 0,1%

La fraction éluée est ensuite diluée dans 3ml de tampon acétate d'ammonium 200mM à pH 3,00.

L'échantillon est fractionné sur une cartouche SPE Waters MCX Oasis selon le protocole suivant :
- La cartouche est conditionnée par 1ml de méthanol puis 1ml de tampon acétate d'ammonium 200mM à pH 3,00.
- Le sérum dilué dans le tampon acétate d'ammonium 200mM à pH 3,00 est déposé en totalité sur la cartouche MCX, puis laissé couler par gravité.
- La cartouche est lavée par 1ml de tampon acétate d'ammonium 200mM à pH 3,00 puis par 1 ml de méthanol 80%, tampon acétate pH 3,00 20%.
- L'élution est réalisée par 1 ml d'un tampon acétate d'ammonium 200mM pH 5,5 /méthanol (50/50).
- L'éluat est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.

L'éluat est ensuite repris dans un mélange 10% d'acétonitrile (ACN)/90% H₂O 0,5% acide formique quantité suffisante pour (QSP) 200µl

Un volume de 100µl de l'échantillon obtenu est injecté et analysé selon les conditions suivantes :
- Chaîne chromatographique Ultimate 3000 de la société Dionex (Sunnyvale, Californie, Etats Unis d'Amérique)
- Colonne Waters Symmetry C18, 2,1mm de diamètre interne, 100mm de long, taille de particule de 3,5µm
- Solvant A : H₂O + 0,1% acide formique
- Solvant B : ACN + 0,1% acide formique

Gradient HPLC défini au **TABLEAU 2** ci-après :

**TABLEAU 2**

| Temps | Débit (µl) | Solvant A (%) | Solvant B (%) |
|---|---|---|---|
| 0 | 300 | 95 | 5 |
| 3 | 300 | 95 | 5 |
| 15 | 300 | 78 | 22 |
| 16 | 300 | 0 | 100 |
| 24 | 300 | 0 | 100 |
| 24,1 | 300 | 95 | 5 |
| 32 | 300 | 95 | 5 |

L'éluat en sortie de colonne chromatographique est directement injecté dans la source d'ionisation du spectromètre de masse de type QTRAP® 5500 de la société Applied Biosystems (Foster City, Etats Unis d'Amérique). Les paramètres machine utilisés sont les suivants :

| | |
|---|---|
| Type de balayage: | MS/MS/MS (MS3) |
| Polarité: | Positive |
| Mode de balayage: | Profile |
| Source d'ionisation: | Turbo V™ (Applied BioSystems) |
| Précurseur: | 636,80 Da |
| Ion 1^{ère} génération: | 472,30 Da |
| Réglage Q1: | Filtrage avec résolution unitaire |
| Réglage Q3: | Trappe ionique linéaire |
| Vitesse de balayage: | 10000 Da/s |
| Piégeage en Q0: | Oui |
| Temps de remplissage de la trappe ionique linéaire en Q3 : | 200,00 ms |
| Tension en entrée de Q3: | 8,00 V |
| Fragmentation: | Oui |
| Temps d'excitation: | 25,00 ms |
| Incrément de balayage de la trappe ionique en Q3 : | 0,12 Da |
| Masse en début du Balayage (Da) : | 500,00 Da |
| Masse en fin de Balayage (Da) : | 850,00 Da |
| temps (s) : | 0,0350 s |
| amplitude des radios fréquences de piégeage début : | 4,30 |
| amplitude des radios fréquences de piégeage fin : | 4,48 |
| Tension de sortie de la trappe à ion (début) | -136,24 V |
| Tension de sortie de la trappe à ion (fin) | -125,09 V |
| Gaz rideau: | 50,00 psi |
| Tension de cône: | 5500,00 V |
| Température de source: | 500,00 °C |
| Gaz de nébulisation: | 50,00 psi |
| Gaz chauffant: | 40,00 psi |
| Remplissage de la cellule de collision: | élevé |
| Potentiel d'orifice ou en anglais declustering potentiel : | 50,00 V |
| Potentiel d'entrée avant Q0: | 3,00 V |
| Energie de collision : | 28,00 e⁻V |
| Energie d'excitation (AF2): | 0,07 |

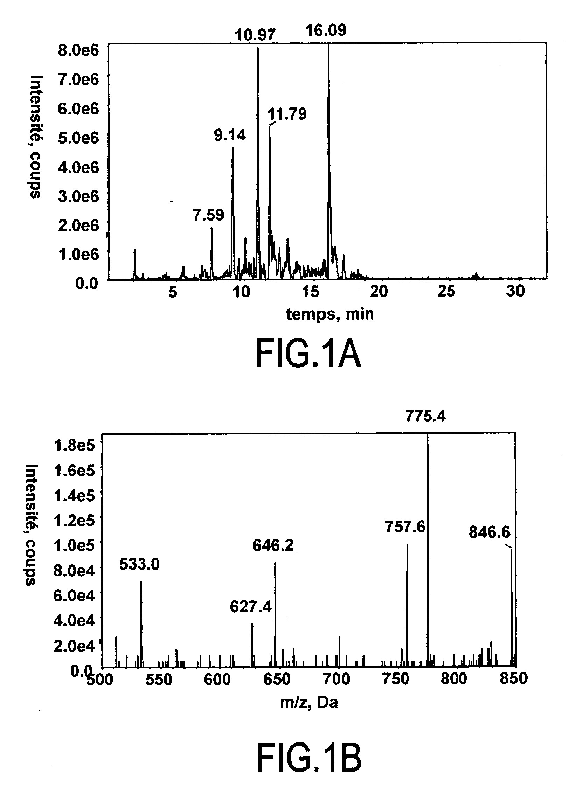

Le signal obtenu est représenté en fonction du temps sous forme d'un chromatogramme d'ion total **(****Figure 1A****).**

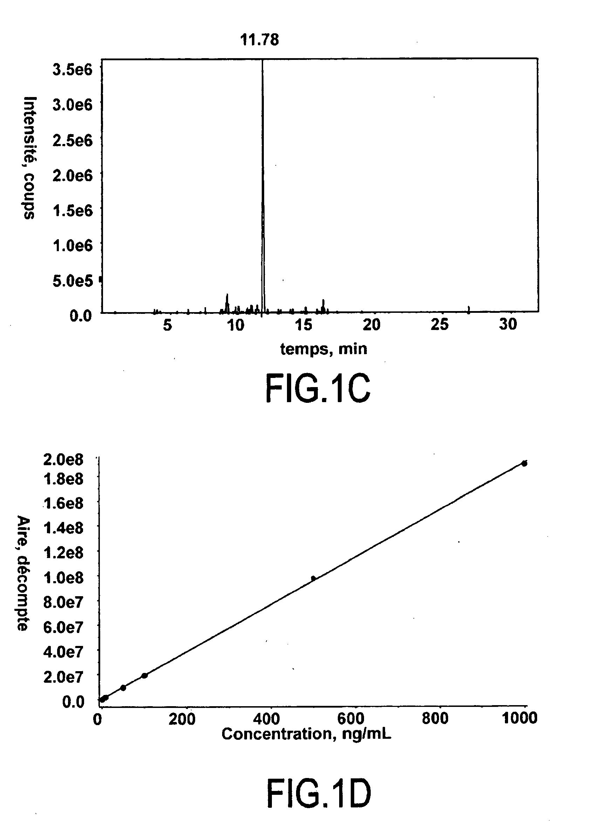

A chaque instant t, les masses d'ion de seconde génération sont observables sous forme d'un spectre de masse. Un spectre de masse mesure donc les masses des différentes espèces éluées de la colonne et injectées simultanément dans le spectromètre de masse. C'est ainsi que les masses d'ion fragment de seconde génération élués entre 11,70 et 11,90 minutes sont présentées sur la **Figure 1****B.** Certaines de ces masses correspondent aux masses de fragments de seconde génération du peptide protéotypique LSEPAELTDAVK **(SEQ ID N°50)** du PSA. C'est ainsi que les masses 533,0, 646,2, 757,6, 775,4 et 846,6 correspondent respectivement aux fragments y5, y6, y7-H₂O, y7 et y8. Comme bien connu dans l'état de la technique, par convention, le fragment « y5 » est un fragment ayant pour séquence les 5 derniers acides aminés de la séquence du peptide protéotypique, le fragment « y6 » les six derniers, etc.

Le logiciel Analyst 1.5 (Applied Biosytems) permet d'extraire le courant ionique correspondant à des fenêtres de masse en fonction du temps. Il permet donc d'obtenir un chromatogramme pour des fenêtres, par exemple d'une unité de masse, correspondant à chaque fragment de seconde génération d'intérêt. Ce chromatogramme est appelé chromatogramme d'ions extraits. Le logiciel Analyst 1.5 permet également de sommer plusieurs chromatogrammes d'ions extraits. Il permet également d'effectuer un lissage du signal selon l'algorithme de Stavitzky et Golay (A. Savitzky and M. Golay (1964). Smoothing and Differentiation of Data by Simplified Least Squares Procedures. Analytical Chemistry, 36: 1627-1639). C'est ainsi que la **Figure 1c** montre la somme des chromatogrammes d'ions extraits correspondants aux masses des fragments de seconde génération y5, y6, y7-H₂O, y7 et y8 du peptide protéotypique LSEPAELTDAVK **(SEQ ID N°50)** du PSA, lissé sur trois points.

Le logiciel Analyst 1.5 permet ensuite d'intégrer l'aire sous les pics observés sur les chromatogrammes d'ions extraits. C'est ainsi que la mesure du signal de la somme des ions y5, y6, y7-H₂O, y7 et y8 du peptide LSEPAELTDAVK **(SEQ ID N°50),** pour les points de gammes ayant une dose de PSA entre 0 et 1000 ng/ml, a permis d'obtenir le **TABLEAU 3** ci-après :

**TABLEAU 3**

| Aire sous les pics | concentration en PSA (ng/ml) |
|---|---|
| 1,25e+005 | 0 |
| 4,99e+005 | 1 |
| 1,50e+006 | 5 |
| 1,62e+006 | 10 |
| 9,42e+006 | 50 |
| 1,90e+007 | 100 |
| 9,73e+007 | 500 |
| 1,90e+008 | 1000 |

Ces résultats permettent d'établir la courbe d'étalonnage représentée **Figure 1D****.** Cette courbe est modélisée à l'aide d'une régression linéaire sous forme d'une équation (y=1,9.10⁵X+2,76.10⁵) permettant de calculer la concentration en PSA pour tout échantillon de sérum humain présentant une dose inconnue de PSA.

A titre d'exemple les sérums des patients suivants sont dosés et permettent d'obtenir les doses suivantes présentées **TABLEAU 4 :**

**TABLEAU 4**

| Patients | Aire sous le pic | concentration calculé (ng/ml) |
|---|---|---|
| B2004 | 1,81E+06 | 8,07 |
| D4003 | 8,48E+05 | 3,01 |
| D4004 | 1,37E+06 | 5,77 |

### Exemple 2 : Principe de dosage quantitatif par MRM³ avec calibration interne d'un marqueur tumoral : l'antigène spécifique de la prostate (PSA)

Un standard de calibration interne est synthétisé en utilisant des acides aminés comportant des isotopes lourds pour certains atomes selon le protocole décrit dans T. Tortin et all (MCP, 2008 E-pub).

C'est ainsi que le peptide organotypique du PSA LSEPAELTDAVK **(SEQ ID N°50)** est synthétisé chimiquement avec 2 alanines comportant chacune 3 carbones 13 (¹³C) à la place des carbones 12 (¹²C). La synthèse est réalisée avec un synthétiseur ABI433A (Applied Biosystems, Foster City, Etats Unis d'Amérique) et de la L-(¹³C₃)Alanine-N-FMOC (Euriso-Top, Saint-Aubin, France). En fin de synthèse, le peptide lourd est reparti en 30 flacons en verre brun de 4 ml, puis lyophilisé.

La pureté du peptide lourd de synthèse est contrôlée par chromatographie couplée avec un analyseur de masse (Trappe ionique LCQ, ThermoFisher Scientific, Waltham, *Massachusetts,* Etats-Unis d'Amérique). Elle est établie comme supérieure à 95%. La quantité de peptide lourd contenu dans les flacons de verre brun est ensuite établie, à partir d'un échantillon de 3 flacons, avec un analyseur d'acides aminés modèle 1100 de la société Agilent Technologies (Massy, France). Il est ainsi déterminé que les flacons contiennent 790 µg de peptide lourd pur à plus de 95%.

Un flacon de 790µg de peptide lourd est repris par 1 ml d'eau additionné de 0,5% d'acide formique. On obtient une solution mère de peptide lourd à une concentration de 6,2.10⁻¹⁰ mol/µl.

La solution mère du peptide lourd est diluée par 10 dans un mélange acétonitrile/eau (50/50) plus 1% acide formique pour obtenir une solution à 6,2.10⁻¹¹ mol/µl

La solution à 6,2.10⁻¹¹ mol/µl est diluée par 10 dans un mélange acétonitrile/eau (50/50) plus 1% acide formique pour obtenir une solution à 6,2.10⁻¹² mol/µl

La solution à 6,2.10⁻¹² mol/µl est diluée par 20 dans un mélange acétonitrile/eau (50/50) plus 1% acide formique pour obtenir une solution à 3,1.10⁻¹³ mol/µl

La solution à 3,1.10⁻¹³ mol/µl est diluée par 10 dans un mélange acétonitrile/eau (50/50) plus 1% acide formique pour obtenir une solution à 3,1.10⁻¹⁴ mol/µl

La solution à 3,1.10⁻¹⁴ mol/µl est diluée par 10 dans un mélange acétonitrile/eau (50/50) plus 1% acide formique pour obtenir une solution à 3,1.10⁻¹⁵ mol/µl

Les échantillons de sérums de patient et les points de gamme sont traités selon le protocole décrit à l'exemple 1 avec ajout d'un volume de 50 µl de la solution de peptide lourd à 3,1.10⁻¹⁵ mol/µl entre l'étape de digestion enzymatique et l'étape de dessalage sur colonne HLB Oasis Waters.

Les échantillons de sérums de patient et les points de gamme sont ensuite analysés selon le protocole décrit à l'exemple 1, avec ajout du suivi des ions correspondant au peptide lourd à savoir :

| | |
|---|---|
| Précurseur: | 639,80 Da |
| Ion 1^{ère} génération: | 475,30 Da |

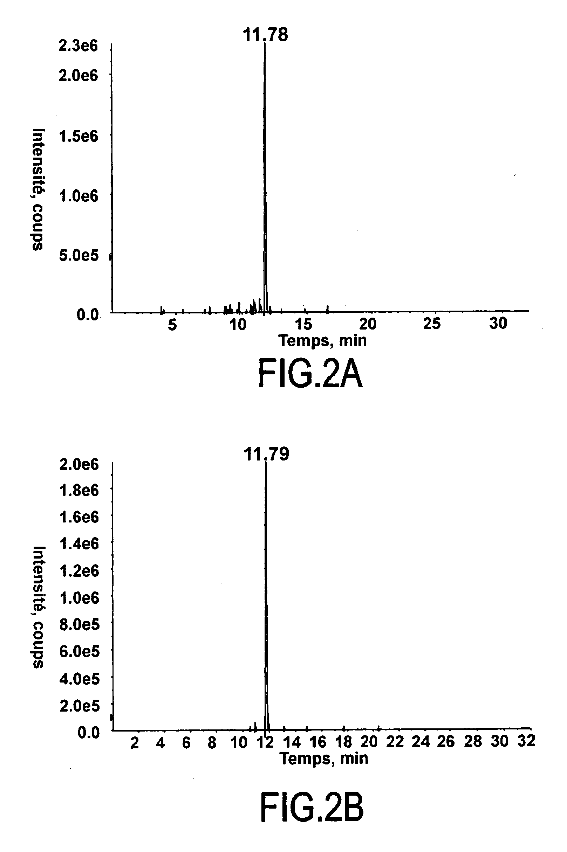

Le peptide lourd LSEPAELTDAVK **(SEQ ID N°50)** a les mêmes propriétés physico-chimiques que le peptide natif, et par conséquent le même temps de rétention en chromatographie. Les deux peptides sont donc injectés simultanément dans le spectromètre de masse. C'est ce qu'il est possible d'observer sur le chromatogramme du peptide LSEPAELTDAVK **(SEQ ID N°50)** natif **Figure 2A** (temps de rétention 11,78 min) et du peptide LSEPAELTDAVK **(SEQ ID N°50)** lourd **Figure 2B** (temps de rétention 11,79 min).

En revanche ce peptide lourd comporte 6 unités de masse de plus que le peptide PSA natif. Il a une masse de 1271,66 g/mol contre 1277,66 g/mol pour le peptide natif.

La quantité de peptide lourd est connue (1,55.10⁻¹³ mol) et est identique dans tous les points de la gamme, le signal reconstitué doit donc être constant tout au long de la gamme. Cependant, une variation du signal peut être observée sur le peptide lourd, ce qui signifie que cette même variation a impacté le signal du peptide natif. Il est donc possible d'utiliser le signal du peptide lourd pour corriger le signal du peptide léger. Pour ce faire, un ratio entre les aires des pics chromatographiques, des ions spécifiques natif et lourd de deuxième génération extraits et sommés, est réalisé avec le logiciel Analyst 1.5.

C'est ainsi que la mesure des signaux des sommes des ions y5, y6, y7-H₂O, y7 et y8 des peptides LSEPAELTDAVK **(SEQ ID N°50)** natif et lourd, pour les points de gammes ayant une dose de PSA entre 0 et 1000 ng/ml, permet d'obtenir le **TABLEAU 5** ci-après:

**TABLEAU 5**

| concentration en PSA (ng/ml) | aire sous le pic du peptide natif | aire sous le pic du peptide lourd |
|---|---|---|
| 0 | 1,25E+05 | 8,11E+06 |
| 1 | 4,99E+05 | 9,42E+06 |
| 5 | 1,50E+06 | 8,96E+06 |
| 10 | 1,62E+06 | 9,11E+06 |
| 50 | 9,42E+06 | 9,80E+06 |
| 100 | 1,90E+07 | 7,48E+06 |
| 500 | 9,73E+07 | 8,89E+06 |
| 1000 | 1,90E+08 | 9,55E+06 |

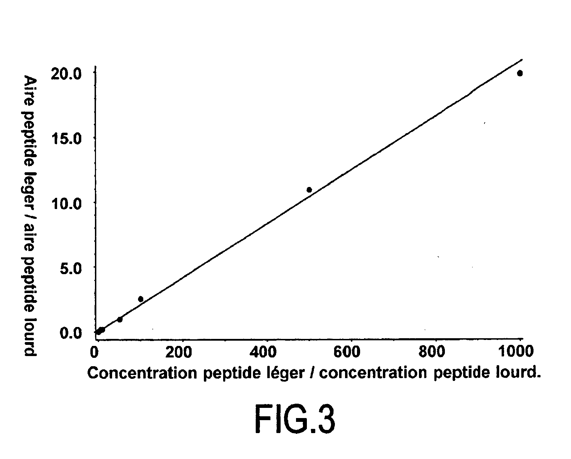

Ces résultats permettent d'établir la courbe d'étalonnage représentée **Figure 3****.** Cette courbe est modélisée à l'aide d'une régression linéaire sous forme d'une équation (y=0,0208(Xnatif/Xlourd)+0,0154) permettant de calculer la concentration en PSA pour tout échantillon de sérum humain présentant une dose inconnue de PSA.

A titre d'exemple, les sérums des patients suivants sont dosés et conduisent aux doses présentées **TABLEAU 6** :

**TABLEAU 6**

| patients | aire sous le pic du peptide natif | aire sous le pic du peptide lourd | concentration calculée (ng/ml) |
|---|---|---|---|
| B2004 | 1,81E+06 | 8,74E+06 | 9,24 |
| D4003 | 8,48E+05 | 9,42E+06 | 3,6 |
| D4004 | 1,37E+06 | 8,26E+06 | 7,27 |

### Exemple 3 : Comparaison des dosages quantitatifs du PSA sérique obtenu par MRM³ avec calibration externe ou interne ou par ELISA

Les sérums de patients, dont le dosage par MRM³ avec calibration externe ou interne a été exposé respectivement dans les exemples 1 et 2, sont dosés par ELISA, d'une part à l'aide de la trousse Vidas® TPSA (bioMérieux, Marcy l'Etoile, France) et de l'automate Vidas®, et d'autre part, à l'aide de la trousse total PSA et de l'automate Modular Analytics E170 de la société Roche Diagnostics (Mannheim, Allemagne). Dans les deux cas, le protocole décrit par le fournisseur est mis en oeuvre. Ces sérums correspondent à des patients atteints d'un cancer de la prostate (PCa) ou d'une hypertrophie bénigne de la prostate (BPH). La comparaison des doses obtenues selon ces 3 méthodes de dosage s'établit comme présentée **TABLEAU 7 :**

**TABLEAU 7**

| Patients | Pathologie | Concentration établie en MRM³ avec calibration externe (ng/ml) | Concentration établie en MRM³ avec calibration interne (ng/ml) | Concentration 1 établie en ELISA avec la trousse Vidas@ TPSA (ng/ml) | Concentration 2 établie en ELISA avec la trousse Total PSA Roche (ng/ml) |
|---|---|---|---|---|---|
| B2004 | BPH | 8,07 | 9,24 | 7,89 | 7,03 |
| D4003 | PCa | 3,01 | 3,6 | 5,72 | 5,2 |
| D4004 | BPH | 5,77 | 7,27 | 8,23 | 7,3 |

Les doses, déterminées à l'aide des méthodes MRM³ avec calibration externe ou interne, conduisent donc à des concentrations en PSA extrêmement proches de celles établies par les méthodes ELISA traditionnelles.

Il faut noter à ce propos que les tests ELISA ne dosent pas toutes les molécules de PSA présentes dans la circulation sanguine. En effet, le PSA forme des complexes avec certaines anti-protéases sanguines comme l'alpha-1 anti-chymotrypsine (ACT) et l'alpha-2 macroglobuline (A2M). Le PSA lié à l'A2M n'est pas détectable par ELISA, alors qu'il est dosable par spectrométrie de masse comme cela a été établi par T. Fortin et al. (MCP, 2008 E-pub). Le complexe PSA-A2M compte pour environ 10% du PSA total, mais cette dose peut être modulée selon la pathologie du patient. Cette propriété particulière du PSA explique une part des écarts de doses observées entre techniques ELISA et MRM³.

### Exemple 4 : Dosage quantitatif de protéines recombinantes de Treponema pallidum diluées dans un sérum humain

Cet exemple est réalisé avec 2 protéines de *Treponema pallidum,* agent infectieux de la syphilis, exprimées de façon recombinante (bioMérieux, Marcy l'Etoile, France). Pour faciliter l'expression in vitro et la purification des protéines recombinantes, la séquence native des protéines de *Treponema pallidum* a été modifiée par le fournisseur. Les séquences exactes des 2 protéines utilisées sont fournies ci-dessous :

### SEQUENCE TP435 SYPHILIS

### SEQUENCE TP574 SYPHILIS

La quantité de protéine recombinante est établie pour Tp435 et Tp574 par dosage de protéine selon la méthode de Bradford (Bradford, M. M. (1976) A Rapid and Sensitive Method for the Quantitation of Microgram Quantities of Protein Utilizing the Principle of Protein-Dye Binding. Anal. Biochem. 72:248-254) à l'aide du réactif « Protein Assay » de la société Bio-Rad (Hercules, Californie, Etats-Unis d'Amérique).

Le lot de protéine Tp435 a ainsi une dose de 0,97 mg/ml. Le lot de protéine Tp574 a ainsi une dose de 1,4 mg/ml.

Les protéines recombinantes TP435 et TP574 sont digérées de la façon suivante :
- Une quantité de 80µg de chaque protéine est prélevée et additionnée de tampon bicarbonate d'ammonium 50mM, pH=8,0 pour obtenir un volume final de 400 µl.
- Ajout de 100µl de DTT 150mM.
- Incubation à 95°C pendant 20 minutes.
- Incubation à 60°C pendant 20minutes.
- Refroidissement à température ambiante des échantillons
- Ajout de 100µl d'iodoacétamide 150mM
- Incubation pendant 40 minutes à température ambiante à l'obscurité.
- Ajout de 4 µg de trypsine
- Incubation pendant 4 heures à 37°C
- En fin de digestion, la concentration de chaque digest de protéine est de 133,3 µg/ml.

Huit volumes de 100 µl de sérum de patients réputés « sains » (Etablissement Français du Sang) sont digérés en parallèle selon le protocole suivant :
- Chaque volume de 100µl de sérum est dilué dans 3ml de bicarbonate d'ammonium 50mM, pH=8,0.
- Ajout du DTT pour obtenir une concentration finale de 15mM.
- Réduction à 60°C pendant 40minutes.
- Refroidissement des tubes à température ambiante.
- Ajout d'iodoacétamide pour obtenir une concentration finale de 25 mM.
- Alkylation pendant 40minutes à température ambiante et à l'obscurité.
- Ajout de trypsine avec un ratio massique de 1/30.
- Digestion à 37°C pendant 4 heures.
- Ajout de nouveau du DTT pour obtenir une concentration finale de 10mM.
- Réduction à 60°C pendant 40minutes.
- Refroidissement des tubes à température ambiante.
- Ajout de nouveau de l'iodoacétamide pour obtenir une concentration finale de 15mM.
- Alkylation à température ambiante et à l'obscurité.
- Ajout de nouveau de la trypsine avec un ratio de 1/30.
- Digestion à 37°C pendant toute la nuit.
- Acidification des échantillons avec de l'acide formique (soit 0,1% final)
- Equilibrage des colonnes HLB Oasis Waters avec 1ml de méthanol, puis 1 ml d'eau « ultrapure »/acide formique 0,1%
- Dépôt de l'échantillon qui est laissé couler par gravité
- Lavage avec 1 ml d'un mélange eau/acide formique 0,1%
- Elution avec 1ml de méthanol 80% dans un mélange eau/acide formique 0,1%
- Séchage des tubes à l'évaporateur de type sSpeedVac® SPD2010 (Thermo Electron Corporation, Waltham, Massachusetts, Etats-Unis d'Amérique) pendant 1 heure jusqu'à l'obtention d'un volume de 500 µl environ.
- Mélange des 8 tubes de sérums traités pour obtenir un pool de sérum digéré homogène.

Réalisation des gammes d'étalonnage :
- 50µl de protéine Tp574 à 133,3 µg/ml est ajouté à 50µl de protéine Tp435 à 133,3 µg/ml. Le tout est ajouté à 400 µl d'eau additionnée de 0,1% d'acide formique pour former une solution mère avec une concentration de 26,6µg/ml pour chacune des protéines Tp574 et Tp435 (solution mère). Un volume de 75µl de la solution mère est ensuite dilué dans 125µl d'eau additionnée de 0,1% d'acide formique pour avoir une solution à 10000ng/ml par protéine.
- 37,5µl de la solution mère sont dilués dans 162,5µl d'eau additionnée de 0,1% d'acide formique pour obtenir une solution à 5000ng/ml
- 20µl de la solution à 10µg/ml sont dilués dans 180µl d'eau pour avoir une solution à 1000 ng/ml
- 20µl de la solution à 1µg/ml sont dilués dans 180µl d'eau pour avoir une solution à 100ng/ml
- 20µl de la solution à 100ng/ml dans 180µl d'eau pour avoir une solution à 10ng/ml
- 20µl de la solution à 5µg/ml sont dilués dans 180µl d'eau pour avoir une solution à 500ng/ml
- 20µl de la solution à 500ng/ml sont dilués dans 180µl d'eau pour avoir une solution à 50ng/ml
- 7 fractions de sérum digéré, correspondant chacune à 100 µl de sérum avant digestion, sont supplémentées avec respectivement 10 µl de chacune des solutions entre 10000 et 50 ng/ml pour obtenir une gamme étalon avec des points à 1, 5, 10, 50, 100, 500 et 1000ng/ml.
- La dernière fraction de sérum est supplémentée avec 10µl d'eau, afin d'obtenir un point de gamme à 0 ng/ml.

Les échantillons et les points de gamme sont analysés avec les paramètres chromatographiques suivants et avec 2 périodes présentant un réglage différent du spectromètre de masse.
- Chaîne chromatographique Ultimate 3000 de la société Dionex (Sunnyvale, Californie, Etats Unis d'Amérique)
- Colonne Waters Symmetry C18, 2,1mm de diamètre interne, 100mm de long, taille de particule de 3,5µm
- Solvant A : H₂O + 0,1% acide formique
- Solvant B : ACN + 0,1% acide formique

Gradient HPLC défini au **TABLEAU 8** ci-après :

**TABLEAU 8**

| Temps | Débit (µl) | Solvant A (%) | Solvant B (%) |
|---|---|---|---|
| 0 | 300 | 95 | 5 |
| 3 | 300 | 95 | 5 |
| 28 | 300 | 60 | 40 |
| 30 | 300 | 0 | 100 |
| 38 | 300 | 0 | 100 |
| 38,1 | 300 | 95 | 5 |
| 48 | 300 | 95 | 5 |

La période 1 correspond au suivi du peptide protéotypique SAPSPLTYR **(SEQ ID N° 53)** de la protéine Tp435. La période 2 correspond au suivi du peptide protéotypique FVPVAVPHELK **(SEQ ID N° 54)** de la protéine Tp574.

Pour chaque peptide protéotypique, des fragments de première génération doublement chargés et comportant une proline en position 1 sont sélectionnés et fragmentés, selon les paramètres machines suivants :

**Période 1** pour la protéine Tp435:

Les paramètres machine sont les mêmes que pour l'exemple 1, à l'exception des paramètres suivants :

| | |
|---|---|
| Précurseur: | 496,50 Da |
| Ion 1^{ère} génération: | 417,50 Da |
| Barrière d'entrée en Q3: | 4,00 V |
| Masse en début du Balayage (Da) : | 430,00 Da |
| Masse en fin de Balayage (Da) : | 750,00 Da |
| temps (s) : | 0,0320 s |
| amplitude des radios fréquences de piégeage début : | 2,88 |
| amplitude des radios fréquences de piégeage fin : | 4,10 |
| Tension de sortie de la trappe à ion (début) | -142,07 V |
| Tension de sortie de la trappe à ion (fin) | -129,13 V |
| Température de source: | 450,00 °C |
| Gaz chauffant: | 50,00 psi |
| Potentiel d'orifice (ou en anglais declustering potentiel) : | 80,00 V |
| Energie de collision : | 18,00 e⁻V |
| Energie d'excitation (AF2): | 0,12 |

**Période 2** la protéine Tp574:

Les paramètres machine sont les mêmes que pour la période 1, à l'exception des paramètres suivants :

| | |
|---|---|
| Précurseur: | 618,40 Da |
| Ion 1^{ère} génération: | 495,50 Da |
| Masse en début du Balayage (Da) : | 500,00 Da |
| Masse en fin de Balayage (Da) : | 850,00 Da |
| temps (s) : | 0,0350 s |
| amplitude des radios fréquences de piégeage début : | 3,15 |
| amplitude des radios fréquences de piégeage fin : | 4,48 |
| Tension de sortie de la trappe à ion (début) | -139,24 V |
| Tension de sortie de la trappe à ion (fin) | -125,09 V |
| Potentiel d'orifice (ou en anglais declustering potentiel) : | 90,00 V |
| Energie de collision : | 25,00 e⁻V |
| Energie d'excitation (AF2): | 0,09 |

La mesure du signal de la somme des ions y3 et y5 (m/z 649.4 et 439.4) du peptide protéotypique SAPSPLTYR **(SEQ ID N°53)** de la protéine TP435, pour les points de gammes ayant une dose entre 5 et 50 ng/ml, a permis d'obtenir le **TABLEAU 9** ci-après :

**TABLEAU 9**

| concentration (ng/ml) | aire du pic | Signal sur bruit | limite de quantification (ng/ml) |
|---|---|---|---|
| 5 | 1,82E+05 | 1 | 28,4 |
| 10 | 1,55E+05 | 1 | |
| 50 | 2,05E+06 | 17,6 | |

La mesure du signal de la somme des ions y5 et y7 (m/z 623.4 et 793.4) du peptide protéotypique FVPVAVPHELK **(SEQ ID N°54)** de la protéine TP 574, pour les points de gammes ayant une dose entre 5 et 50 ng/ml, a permis d'obtenir le **TABLEAU 10** ci-après :

**TABLEAU 10**

| concentration (ng/ml) | aire du pic | Signal sur bruit | limite de quantification (ng/ml) |
|---|---|---|---|
| 5 | 6,14E+05 | 6,3 | 8,7 |
| 10 | 2,58E+06 | 11,5 | |
| 50 | 1,92E+07 | 61,9 | |

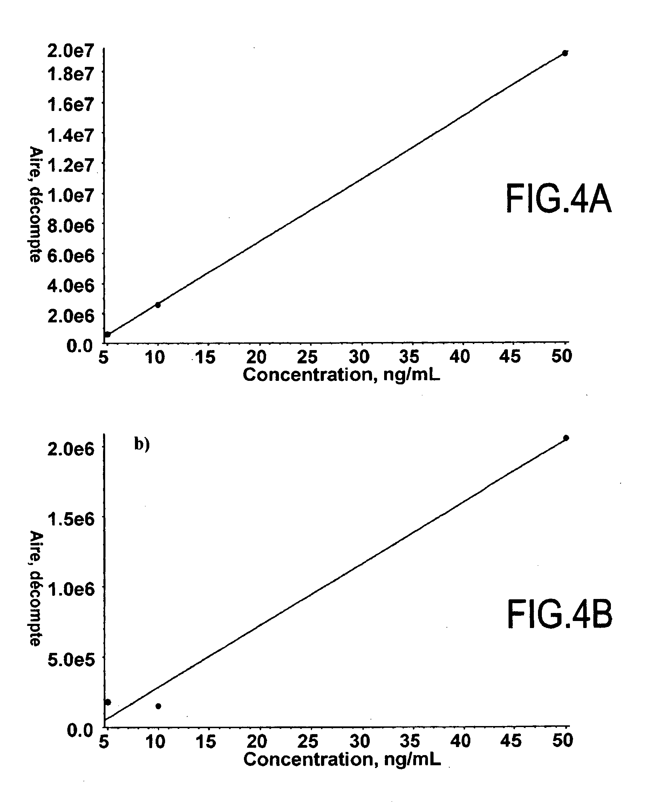

Ces résultats permettent d'établir les courbes d'étalonnage pour les protéines Tp435 et Tp574 respectivement représentées sur les **Figures 4A** et **4B****.**

Ces courbes sont modélisées à l'aide de régression linéaire sous forme d'équations permettant de calculer la concentration en Tp435 et Tp574 pour tout échantillon de sérum humain présentant une dose inconnue de Tp435 ou de Tp574.

La concentration de Tp435 se calcule grâce à l'équation : y= 4,14.10⁵ X-1,5.10⁶

La concentration de Tp574 se calcule grâce à l'équation : y= 4,38.10⁴ X-1,5.10⁵

### Exemple 5 : Amélioration des performances des dosages quantitatifs avec la méthode MRM³ utilisant des ions dichargés comme fragment de première génération

Les performances analytiques obtenues à l'exemple 4 sont comparées, dans les mêmes conditions de traitement de l'échantillon, aux performances obtenues avec un dosage MRM traditionnel ou avec un dosage MRM³ n'utilisant pas de fragment de première génération dichargé. Pour chaque protéine, le même peptide protéotypique est dosé par les trois méthodes, à savoir le peptide LSEPAELTDAVK **(SEQ ID N°50)** pour le PSA, le peptide SAPSPLTYR **(SEQ ID N°53)** pour la protéine Tp435 et le peptide FVPVAVPHELK **(SEQ ID N°54)** pour la protéine Tp574.

L'analyse est réalisée avec des échantillons préparés selon le protocole de l'exemple 4 avec une solution mère comportant 50µl de protéine Tp574 à 133,3 µg/ml, plus 50µl de protéine Tp435 à 133,3 µg/ml, plus 50 µl de protéine PSA à 133,3 µg/ml, plus 350 µl d'eau additionnée de 0,1% d'acide formique.

L'analyse en MRM³ avec des fragments de première génération dichargés est réalisée avec la méthode de chromatographie et de spectrométrie de masse décrite à l'exemple 4 pour les trois protéines. Les réglages du spectromètre de masse sont établis sur 3 périodes correspondant aux réglages adaptées à chaque peptide protéotypique. Ce dosage est appelé procédé 1 pour cet exemple.

L'analyse en MRM³ avec des fragments de première génération monochargé est réalisée avec les ions y9 (943,5m/z) pour le peptide protéotypique du PSA de m/z 636,8, (y5, 649,2m/z) pour le peptide protéotypique de Tp435 de m/z 496,8 et (y5, 623,3m/z) pour le peptide protéotypique de Tp574 de m/z 618,8. Le pic chromatographique est obtenu en sommant le signal des fragments de deuxième génération de m/z 609+627+646+698 pour le peptide protéotypique du PSA, 631,2+457,4+475,4 pour le peptide protéotypique de Tp435 et 477,5+364,2+605,4 pour le peptide protéotypique de Tp574. Ce dosage est appelé procédé 2 pour cet exemple.

Pour le procédé 2 le fonctionnement du spectromètre de masse est divisé en 3 périodes.

**Période 1** pour le PSA:

Les paramètres machine sont les mêmes que pour l'exemple 1, à l'exception des paramètres suivants :

| | |
|---|---|
| Précurseur: | 636,80 Da |
| Ion 1^{ère} génération: | 943,50 Da |
| Masse en début du Balayage (Da) : | 600,00 Da |
| Masse en fin de Balayage (Da) : | 800,00 Da |
| temps (s) : | 0,0200 s |
| amplitude des radios fréquences de piégeage début : | 3,53 |
| amplitude des radios fréquences de piégeage fin : | 4,29 |
| Tension de sortie de la trappe à ion (début) | -135,20 V |
| Tension de sortie de la trappe à ion (fin) | -127,11 V |
| Température de source: | 450,00 °C |
| Gaz chauffant: | 50,00 psi |
| Potentiel d'orifice (ou en anglais declustering potentiel) : | 120,00 V |
| Potentiel d'entrée avant Q0: | 4,00 V |
| Energie de collision : | 23,00 e⁻V |
| Energie d'excitation (AF2): | 0,12 |

**Période 2** pour la protéine TP435 :

Les paramètres machine sont les mêmes que pour la période 1, à l'exception des paramètres suivants :

| | |
|---|---|
| Précurseur: | 496,50 Da |
| Ion 1^{ère} génération : | 649,20 Da |
| Barrière d'entrée en Q3: | 4,00 V |
| Masse en début du Balayage (Da) : | 430,00 Da |
| Masse en fin de Balayage (Da) : | 640,00 Da |
| temps (s) : | 0,0210 s |
| amplitude des radios fréquences de piégeage début : | 2,88 |
| amplitude des radios fréquences de piégeage fin : | 3,68 |
| Tension de sortie de la trappe à ion (début) | -142,07 V |
| Tension de sortie de la trappe à ion (fin) | -133,58 V |
| Potentiel d'orifice (ou en anglais « declustering potentiel | ») : 80,00 V |
| Energie de collision : | 23,00 e⁻V |
| Energie d'excitation (AF2): | 0,12 |

**Période 3** pour la protéine Tp574:

Les paramètres machine sont les mêmes que pour la période 1, à l'exception des paramètres suivants :

| | |
|---|---|
| Précurseur: | 618,40 Da |
| Ion 1^{ère} génération: | 623,30 Da |
| Barrière d'entrée en Q3: | 4,00 V |
| Masse en début du Balayage (Da) : | 350,00 Da |
| Masse en fin de Balayage (Da) : | 620,00 Da |
| temps (s) : | 0,0270 s |
| amplitude des radios fréquences de piégeage début : | 2,58 |
| amplitude des radios fréquences de piégeage fin : | 3,60 |
| Tension de sortie de la trappe à ion (début) | -145,30 V |
| Tension de sortie de la trappe à ion (fin) | -134,39 V |
| Energie de collision : | 35,00 e⁻V |
| Energie d'excitation (AF2): | 0,10 |

L'analyse en MRM est réalisée avec les ions y9 (943,5m/z) pour le peptide protéotypique du PSA de m/z 636,8, (y5, 649,2m/z) pour le peptide protéotypique de Tp435 de m/z 496,8 et (y5, 623,3m/z) pour le peptide protéotypique de Tp574 de m/z 618,8. Ce dosage est appelé procédé 3 pour cet exemple.

Pour le procédé 3 les paramètres du spectromètre de masse sont les suivant :

| | |
|---|---|
| Type de balayage: | MRM (MRM) |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause entre deux balayages: | 5,007ms |
| Q1 Mass (Da) | 496,20 |
| Q3 Mass (Da) | 649,30 |
| Temps de balayage | 30,00 |
| Potentiel d'orifice | 110V |
| Energie de collision | 25 e-V |
| Potentiel de sortie de cellule de collision | 10 V |
| | |
| Q1 Mass (Da) | 636,8 |
| Q3 Mass (Da) | 943,5 |
| Temps de balayage | 35,00 |
| Potentiel d'orifice | 115V |
| Energie de collision | 23e-V |
| Potentiel de sortie de cellule de collision | 22 V |
| | |
| Q1 Mass (Da) | 618,4 |
| Q3 Mass (Da) | 623,4 |
| Temps de balayage | 35,00 |
| Potentiel d'orifice | 120V |
| Energie de collision | 29 e-V |
| Potentiel de sortie de cellule de collision | 10 V |
| | |
| Gaz rideau: | 50,00 psi |
| Tension de cône: | 5500,00 V |
| Température de source: | 500,00 °C |
| Gaz de nébulisation: | 50,00 psi |
| Gaz chauffant: . | 40,00 psi |
| Remplissage de la cellule de collision | 9,00 (unité arbitraire) |
| Epotentiel d'entrée avant Q0 | 5,00 V |

La limite de quantification des 3 protéines est déterminée en calculant la dose de protéine pour laquelle un ratio du signal divisé par le bruit de fond de 10 est obtenu, comme présenté **TABLEAU 11.**

**TABLEAU 11**

| | procédé 1 | | procédé 2 | | procédé 3 | |
|---|---|---|---|---|---|---|
| Protéines | Signal sur bruit du pic à 50ng/ ml | limite de quantification (en ng/ml) sur ion première génération dichargé | Signal sur bruit du pic à 50ng/ml | limite de quantification (en ng/ml) sur ion première génération monochargé | Signal sur bruit du pic à 50ng/ ml | limite de quantification (en ng/ml) en MRM |
| TP574 | 57,5 | 8,7 | 6,4 | 78,1 | 3,2 | 156,3 |
| TP435 | 17,6 | 28,4 | 4,3 | 116,3 | 11,9 | 42,0 |
| PSA | 34,8 | 14,4 | 16,1 | 31,1 | 19,4 | 25,8 |

Les limites de quantifications obtenues avec le procédé décrit dans la présente invention sont nettement plus basses que celles obtenues avec les procédés 2 et 3. La présente invention conduit donc à un dosage quantitatif plus sensible que les autres procédés.

### Exemple 6 : Optimisation de l'énergie de collision

Le choix de l'ion précurseur et du fragment de première génération pour réaliser la MRM³ est primordial. Il ne faut pas nécessairement choisir le fragment de première génération le plus intense. Dans le cadre de l'invention, il a été mis en évidence que les ions dichargés ayant une proline ou une histidine présentent une fragmentation plus spécifique et génèrent moins de fragmentations secondaires en MS³ et qu'ils sont beaucoup plus adaptés pour obtenir le dosage quantitatif le plus performant.

A titre d'exemple, l'énergie de collision du peptide protéotypique SAPSPLTYR **(SEQ ID N°53)** de la protéine Tp435 a été optimisée.

Un volume de 50 µl de la protéine Tp435 est digéré, dessalé sur HLB Oasis Waters et ajouté à 300µl d'un mélange ACN/eau 50/50 plus 0,1% d'acide formique. Le mélange est ensuite infusé dans le spectromètre de masse avec un débit de 10µl/minute.

Les paramètres machines sont les suivants :

| | |
|---|---|
| Type de balayage: | Magnification des ions produits (ou en anglais Enhanced Product Ion ou EPI) |
| Polarité: | Positive |
| Mode de balayage: | Profile |
| Source d'ionisation: | Turbo V (Applied Biosystems) |
| Précuseur: | 496,20 Da |
| Resolution en Q1: | Unitaire |
| Vitesse de balayage: | 10000 Da/s |
| Piégeage en Q0: | Non |
| Temps de remplissage de la trappe ionique linéaire en Q3 : | 1,00 ms |
| Remplissage dynamique: | Actif |
| TIC Target EMS Scan: | 10,00 .10⁷ coups. |
| Cible de courant total (en anglais TIC Target) : | 10,00 .10⁷ coups. |
| Temps maximum de remplissage: | 250,000 ms |
| Temps minimum de remplissage | 0,050 ms |
| Temps de remplissage par défaut : | 1,000 ms |
| Tension en entrée de Q3: | 8,00 V |
| Incrément de balayage de la trappe ionique en Q3: | 0,12 Da |
| Masse en Début du Balayage (Da) : | 200,00 Da |
| Masse en fin de Balayage (Da) : | 667,01 Da |
| temps (s) : | 0,0467 s |
| amplitude des radios fréquences de piégeage début : | 2,19 |
| amplitude des radios fréquences de piégeage fin : | 3,82 |
| Tension de sortie de la trappe à ion (début) : | -149,35 V |
| Tension de sortie de la trappe à ion (fin) : | -125,68 V |
| Masse en Début du Balayage (Da) : | 667,01 Da |
| Masse en fin de Balayage (Da) : | 1000 Da |
| temps (s) : | 0,0333 s |
| amplitude des radios fréquences de piégeage début : | 3,82 |
| amplitude des radios fréquences de piégeage fin : | 4,99 |
| Tension de sortie de la trappe à ion (début) : | -125,68 V |
| Tension de sortie de la trappe à ion (fin) : | -108,80 V |
| Gaz rideau: | 30,00 psi |
| Tension de cône: | 5500,00 V |
| Température de source: | température ambiante |
| Gaz de nébulisation : | 18,00 psi |
| Gaz chauffant: | température ambiante |
| Remplissage de la cellule de collision: | élevé |
| Potentiel d'orifice : | 100,00 V |
| Potentiel d'entrée avant Q0: | 10,00 V |
| Energie de collision : | entre 5 et 60 e⁻V |

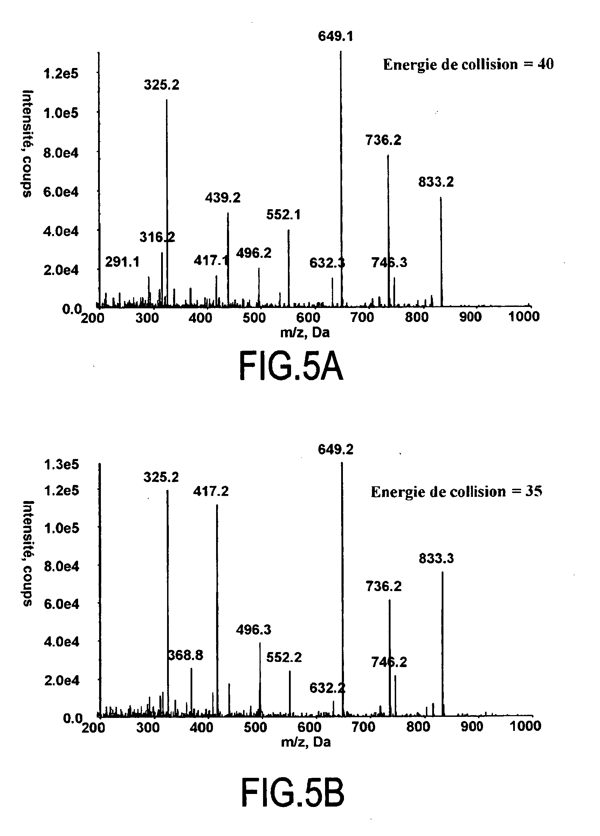

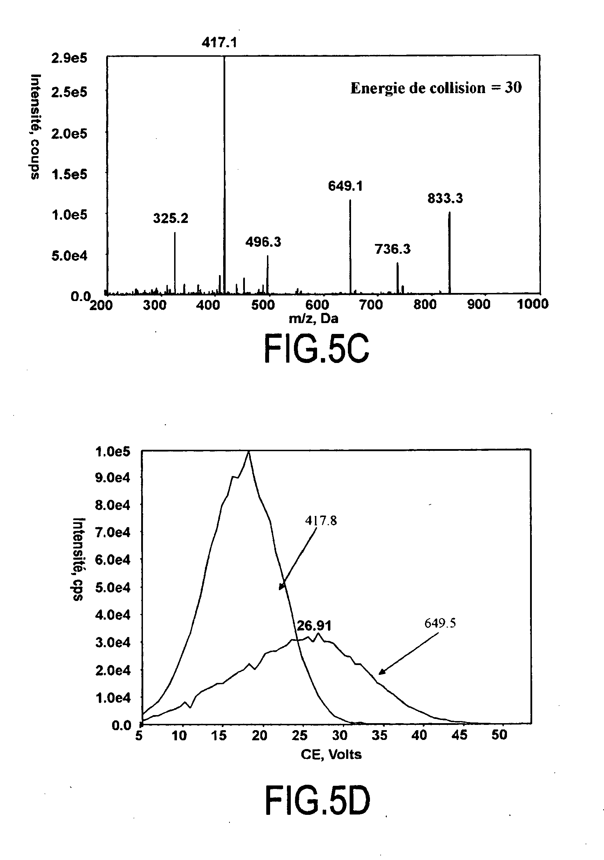

En paramétrant l'énergie de collision à 40 e⁻V **(****Figure 5A****),** on observe que le fragment 417,2, correspondant au fragment de première génération dichargé contenant une proline en N terminale, est peu intense comparé à une fragmentation avec une énergie de collision de 35 ou de 30 e⁻V (respectivement **Figures 5B** et **5C****).** Lorsque l'intensité du fragment de première génération 417,2 diminue, l'intensité des autres fragments augmente. Il est donc tentant d'utiliser une énergie de collision de 40 e⁻V en choisissant le fragment de première génération le plus intense, c'est à dire celui de 649,5 Da correspondant à un fragment de première génération monochargé. Ce choix n'est cependant pas optimum comme le montre l'optimisation de l'énergie de collision visible **Figure 5D****.**

L'optimisation de l'énergie de collision est effectuée en infusion à 10µl/min et en faisant varier uniquement l'énergie de collision de 5 à 60eV. La seule différence par rapport aux paramètres précédents est l'utilisation d'un mode de balayage de type MRM avec une sélection de la masse 496,5 Da en Q1 et des masses 417,8 ou 649,5 en Q3 avec un temps de cycle de 100s, ainsi que l'ajustement suivant des paramètres :

| | |
|---|---|
| Gaz de nébulisation : | 35,00 |
| Potentiel d'orifice : | 80,00 |
| Potentiel d'entrée avant Q0: | 3,00 |

L'optimisation de l'énergie de collision des transitions 496,3/417,8, correspondant au fragment dichargé contenant une proline en N terminale, et 496,3/649,5, correspondant au fragment monochargé le plus intense montre sur la **Figure 5D** que l'énergie de collision permet d'obtenir un gain en signal significatif avec la transition 496,3/417,8, selon un dosage correspondant à l'exemple 4.

Ce gain en signal se traduit par un dosage quantitatif présentant de meilleures performances analytiques, caractérisées par une limite de quantification plus basse, comme le montre le **TABLEAU 12.**

**TABLEAU 12**

| Protéine | limite de quantification (ng/ml) avec l'ion de première génération monochargé | limite de quantification (ng/ml) avec l'ion de première génération dichargé |
|---|---|---|
| TP574 | 78,1 | 8,7 |

Après optimisation des paramètres de spectrométrie de masse, la limite de quantification obtenue avec l'ion de première génération dichargé, selon le procédé décrit dans la présente invention, est nettement plus basse que celle obtenue avec l'ion de première génération monochargé. La méthode utilisant l'ion de première génération dichargé permet donc d'obtenir une méthode de quantification plus sensible.

### Exemple 7 : Amélioration des performances des dosages quantitatifs avec la méthode MRM³ utilisant des ions dichargés comme fragment de première génération

La fragmentation du peptide protéotypique SAPSPLTYR **(SEQ ID N°53)** de la protéine Tp435, qui présente deux prolines dont une en position 2, a été comparée en choisissant comme ion de première génération, soit l'ion fragment le plus intense, soit l'ion fragment contenant une proline sous forme monochargée, soit ce même ion sous forme dichargée.

L'ion de première génération monochargé le plus intense est l'ion y5 de m/z 649,2. L'ion fragment de première génération contenant une proline sous forme monochargée ou dichargée est l'ion y7, respectivement de m/z 833,4 pour la forme monochargée et de m/z 417,5 pour la forme dichargée.

Un volume de 50 µl de la protéine Tp435 est digéré, dessalé sur HLB Oasis Waters et ajouté à 300µl d'un mélange ACN/eau 50/50 plus 0,1% d'acide formique. Le mélange est ensuite infusé dans le spectromètre de masse avec un débit de 10µl/minute.

Les paramètres machines sont les suivants :
Pour la MS³ de l'ion de première génération monochargé le plus intense :

| | |
|---|---|
| Type de balayage: | MS³ |
| Polarité: | Positive |
| Mode de balayage: | Profile |
| Source d'ionisation: | Turbo V (Applied Biosystems) |
| Précurseur: | 496,30 Da |
| Ion 1^{ère} génération : | 649,30 Da |
| Résolution en Q1: | Unitaire |
| Vitesse de balayage: | 10000 Da/s |
| Piégeage en Q0: | Oui |
| Temps de remplissage de la trappe ionique linéaire en Q3 : | 150,00 ms |
| Remplissage dynamique: | Non |
| Fragmentation: | Oui |
| Temps d'excitation : | 25,00 ms |
| Tension en entrée de Q3: | 8,00 V |
| Incrément de balayage de la trappe ionique en Q3: | 0,12 Da |
| Masse en Début du Balayage (Da) : | 250,00 Da |
| Masse en fin de Balayage (Da) : | 640,00 Da |
| Amplitude des radios fréquences de piégeage début : | 3,08 |
| Amplitude des radios fréquences de piégeage fin : | 4,36 |
| Tension de sortie de la trappe à ion (début) : | -144,99 V |
| Tension de sortie de la trappe à ion (fin) : | -122,19 V |
| Gaz rideau: | 20,00 psi |
| Tension de cône: | 5500,00 V |
| Température de source: | température ambiante |
| Gaz de nébulisation : | 35,00 psi |
| Gaz chauffant: | température ambiante |
| Remplissage de la cellule de collision: | élevé |
| Potentiel d'orifice : | 110,00 V |
| Potentiel d'entrée avant Q0: | 10,00 V |
| Energie de collision : | 24 e⁻V |
| Energie d'excitation : | 0,11 e⁻V |

Pour la MS³ de l'ion de première génération monochargé y7 :

| | |
|---|---|
| Type de balayage: | MS³ |
| Polarité: | Positive |
| Mode de balayage: | Profile |
| Source d'ionisation: | Turbo V (Applied Biosystems) |
| Précurseur: | 496,30 Da |
| Ion 1^{ère} génération : | 833,40 Da |
| Résolution en Q1: | Unitaire |
| Vitesse de balayage: | 10000 Da/s |
| Piégeage en Q0: | Oui |
| Temps de remplissage de la trappe ionique linéaire en Q3 : | 150,00 ms |
| Remplissage dynamique: | Non |
| Fragmentation: | Oui |
| Temps d'excitation : | 25,00 ms |
| Tension en entrée de Q3: | 8,00 V |
| Incrément de balayage de la trappe ionique en Q3: | 0,12 Da |
| Masse en Début du Balayage (Da) : | 300,00 Da |
| Masse en fin de Balayage (Da) : | 820,00 Da |
| Amplitude des radios fréquences de piégeage début : | 3,24 |
| Amplitude des radios fréquences de piégeage fin : | 4,95 |
| Tension de sortie de la trappe à ion (début) : | -142,06 V |
| Tension de sortie de la trappe à ion (fin) : | -111,66 V |
| Gaz rideau: | 20,00 psi |
| Tension de cône: | 5500,00 V |
| Température de source: | température ambiante |
| Gaz de nébulisation : | 35,00 psi |
| Gaz chauffant: | température ambiante |
| Remplissage de la cellule de collision: | élevé |
| Potentiel d'orifice : | 110,00 V |
| Potentiel d'entrée avant Q0: | 10,00 V |
| Energie de collision : | 24 e⁻V |
| Energie d'excitation : | 0,14 e⁻V |

Pour la MS³ de l'ion de première génération dichargé y7 :

| | |
|---|---|
| Type de balayage: | MS³ |
| Polarité: | Positive |
| Mode de balayage: | Profile |
| Source d'ionisation: | Turbo V (Applied Biosystems) |
| Précurseur: | 496,30 Da |
| Ion 1^{ère} génération : | 417,50 Da |
| Résolution en Q1: | Unitaire |
| Vitesse de balayage: | 10000 Da/s |
| Piégeage en Q0: | Oui |
| Temps de remplissage de la trappe ionique linéaire en Q3 : | 150,00 ms |
| Remplissage dynamique: | Non |
| Fragmentation: | Oui |
| Temps d'excitation : | 25,00 ms |
| Tension en entrée de Q3: | 8,00 V |
| Incrément de balayage de la trappe ionique en Q3: | 0,12 Da |
| Masse en Début du Balayage (Da) : | 300,00 Da |
| Masse en fin de Balayage (Da) : | 850,00 Da |
| Amplitude des radios fréquences de piégeage début : | 3,24 |
| Amplitude des radios fréquences de piégeage fin : | 5,05 |
| Tension de sortie de la trappe à ion (début) : | -142,06 V |
| Tension de sortie de la trappe à ion (fin) : | -109,91 V |
| Gaz rideau: | 20,00 psi |
| Tension de cône: | 5500,00 V |
| Température de source: | température ambiante |
| Gaz de nébulisation : | 35,00 psi |
| Gaz chauffant: | température ambiante |
| Remplissage de la cellule de collision: | élevé |
| Potentiel d'orifice : | 110,00 V |
| Potentiel d'entrée avant Q0: | 10,00 V |
| Energie de collision : | 25 e⁻V |
| Energie d'excitation : | 0,12 e⁻V |

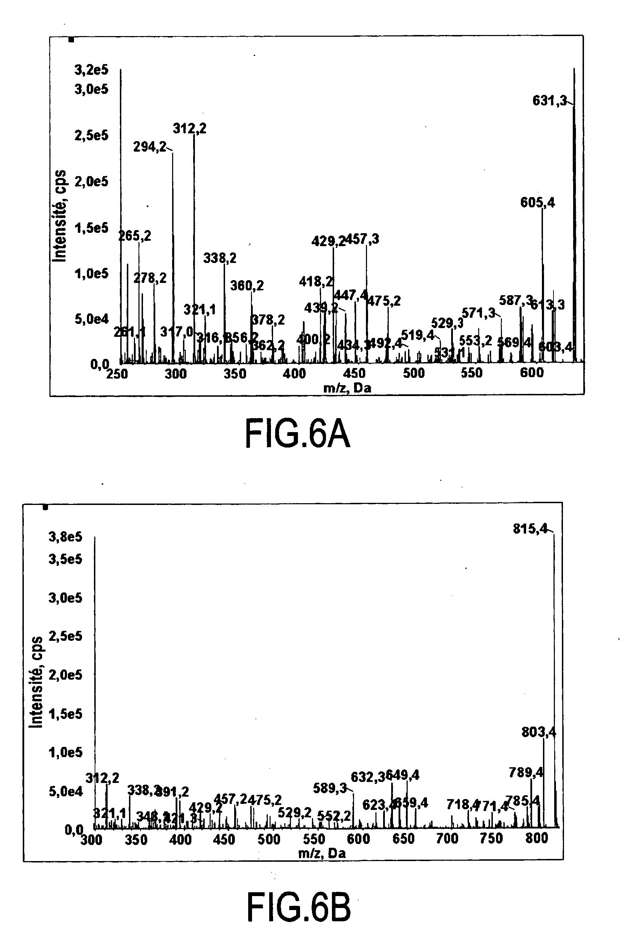

Pour l'ion fragment monochargé de première génération le plus intense, de m/z égale à 649,2, la fragmentation obtenue en MS³ est, comme dans le cas précédent, très complexe, divisant le signal entre tous les pics de fragmentation secondaire, comme montré sur la **Figure 6A****.** Le pic le plus intense du spectre de MS³ correspond à 3,2.10⁵ coups.

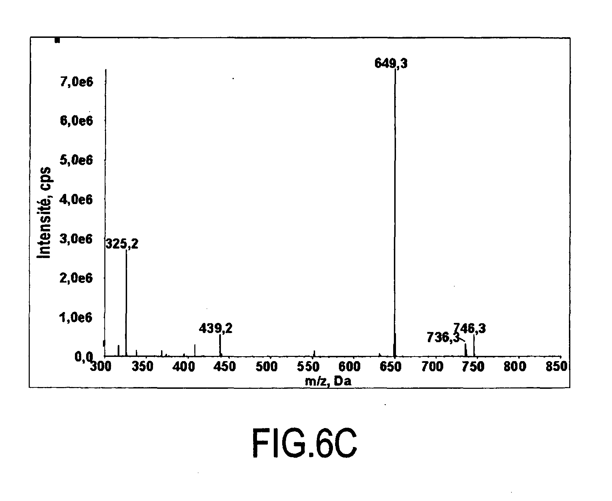

Pour l'ion fragment de première génération monochargé y7, de m/z égale à 833,4, le spectre de fragmentation observé est très complexe, contenant beaucoup de pics de fragmentation secondaire, comme montré sur la **Figure 6****B.** Le pic le plus intense du spectre de MS³ correspond à 3,8.10⁵ coups.

Pour l'ion de première génération dichargé y7, de m/z égale à 417,5, la fragmentation obtenue est beaucoup plus simple et le signal est concentré sur 5 pics majoritaires, comme montré sur la **Figure 6C****.** Le signal du pic majoritaire est de 7,3.10⁶ coups.

Ceci montre que le choix du peptide selon la présente invention conduit à un dosage plus sensible et explique le gain en sensibilité observé dans l'exemple 5.

Le choix du fragment de première génération pour réaliser la MRM³ est primordial. Comme montré dans cet exemple, il ne faut pas nécessairement choisir le fragment de première génération le plus intense. Le choix de l'ion précurseur est également important. Dans le cadre de l'invention, il a été mis en évidence que les ions précurseurs doublement chargés ayant deux prolines ou une proline et une histidine présentent une fragmentation plus spécifique et génèrent encore moins de fragmentations secondaires en MS³ et qu'ils sont beaucoup plus adaptés pour obtenir le dosage quantitatif le plus performant.

## Revendications

1. Procédé pour détecter, de manière quantitative, une protéine cible dans un échantillon comprenant les étapes suivantes :
a) le traitement de l'échantillon pour générer des peptides,
b) le dosage quantitatif d'au moins un peptide protéotypique généré à partir de la protéine cible, par une technique de spectrométrie de masse dans laquelle :
i) le peptide protéotypique est ionisé en ions précurseurs, qui sont filtrés en fonction de leur masse m/z, et un ion précurseur de masse (m/z)₁ donné est sélectionné en fonction de la protéine cible recherchée,
ii) l'ion précurseur sélectionné est fragmenté en ions fragments de première génération,
iii) les ions fragments de première génération générés sont filtrés en fonction de leur masse m/z et un ion fragment de première génération de masse (m/z)₂ donné est sélectionné en fonction de la protéine cible recherchée,
iv) l'ion fragment de première génération sélectionné est fragmenté en ions fragments de deuxième génération,
v) au moins une partie des ions fragments de seconde génération sont détectés pour donner une série de mesures quantitatives,
vi) au moins une mesure quantitative associée à un ion de seconde génération est sélectionnée et corrélée à la quantité du peptide protéotypique généré et à la quantité de protéine cible présente dans l'échantillon,
**caractérisé en ce que** l'ion fragment de première génération de masse (m/z)₂ sélectionné est un peptide doublement chargé qui présente une proline et/ou une histidine en position 1.

2. Procédé selon la revendication 1 **caractérisé en ce que** l'ion précurseur de masse (m/z)₁ sélectionné est un peptide doublement chargé qui contient un nombre n de 6 à 15 acides aminés, et qui présente au moins une proline au niveau des positions 2 à n-2 et/ou une histidine au niveau des positions 1 à n-2.

3. Procédé selon l'une des revendications précédentes, **caractérisé en ce que** le dosage quantitatif des peptides générés par spectrométrie de masse est précédé d'une séparation par chromatographie ou électrophorèse des peptides générés à l'étape a).

4. Procédé selon la revendication 3 **caractérisé en ce que** la séparation par chromatographie utilise une séparation par chromatographie en phase inverse.

5. Procédé selon l'une des revendications précédentes **caractérisé en ce que** le dosage quantitatif des peptides générés par spectrométrie de masse est effectué avec un spectromètre de masse comprenant un triple quadripôle.

6. Procédé selon l'une des revendications précédentes **caractérisé en ce que** le dosage quantitatif des peptides générés par spectrométrie de masse est effectué avec un spectromètre de masse comprenant une trappe ionique.

7. Procédé selon l'une des revendications précédentes **caractérisé en ce que** le traitement de l'échantillon est réalisé par digestion avec une enzyme protéase, par exemple la trypsine.

8. Procédé selon l'une des revendications précédentes **caractérisé en ce qu'**à l'étape v) l'intensité du courant induit par au moins une partie des ions fragments de deuxième génération est détectée en fonction du temps, et le signal obtenu sur une période donnée est décomposé en un spectre de masse des différents ions présents en fonction de leur masse m/z, de manière à obtenir un pic de masse associé à chacun des ions de seconde génération détectés présents sur la période donnée, et le signal correspondant au courant d'au moins un ion de deuxième génération sélectionné est recomposé, et l'intensité du courant correspondant mesurée est la mesure quantitative sélectionné à l'étape vi).

9. Procédé selon la revendication 8 **caractérisé en ce qu'**à l'étape vi), on sélectionne la mesure quantitative associée à l'ion fragment de deuxième génération, présentant le pic m/z le plus intense sur la période donnée.

10. Procédé selon la revendication 8 ou 9 **caractérisé en ce qu'**à l'étape vi), la corrélation est effectuée à partir de la somme d'au moins deux mesures quantitatives, chacune étant associée aux ions fragments de deuxième génération présentant les pics m/z les plus intenses sur la période donnée.

11. Procédé selon l'une des revendications précédentes **caractérisés en ce que** la corrélation réalisée à l'étape vi) est effectuée grâce à une courbe de calibration.

12. Procédé selon l'une des revendications 1 à 10 **caractérisé en ce que** la corrélation réalisée à l'étape vi) est effectuée par calibrage interne avec un peptide lourd.

## Patentansprüche

1. Verfahren zum quantitativen Nachweis eines Zielproteins in einer Probe, umfassend die folgenden Schritte:
a) Behandlung der Probe zur Erzeugung von Peptiden,
b) quantitative Analyse wenigstens eines proteotypischen Peptids, das aus dem Zielprotein erzeugt wurde, durch eine Massenspektrometrietechnik bei der:
i) das proteotypische Peptid zu Vorläuferionen ionisiert wird, welche gemäß ihrer Masse m/z filtriert werden und ein egebenes Vorläuferion der Masse (m/z)₁ gemäß des gesuchten Zielproteins ausgewählt wird,
ii) das ausgewählte Vorläuferion in Ionenfragmente der ersten Generation fragmentiert wird,
iii) die erzeugten Ionenfragmente der ersten Generation gemäß ihrer Masse m/z filtriert werden und ein gegebenes lonenfragment der ersten Generation der Masse (m/z)₂ gemäß des gesuchten Zielproteins ausgewählt wird,
iv) das ausgewählte lonenfragment der ersten Generation in lonenfragmente der zweiten Generation fragmentiert wird,
v) wenigstens ein Teil der Ionenfragmente der zweiten Generation nachgewiesen wird, um eine Reihe quantitativer Messungen zu ergeben,
vi) wenigstens eine quantitative Messung in Verbindung mit einem Ion der zweiten Generation ausgewählt wird und der Menge des erzeugten proteotypischen Peptids und der Menge des in der Probe befindlichen Zielproteins zugeordnet wird,
**dadurch gekennzeichnet, dass** das ausgewählte Ionenfragment der ersten Generation der Masse (M/Z)₂ ein doppelt-geladenes Peptid ist, das ein Prolin und/oder ein Histidin an Position 1 aufweist.

2. Verfahren nach Anspruch 1, **dadurch gekennzeichnet, dass** das ausgewählte Vorläuferion der Masse (m/z)₁ ein doppelt-geladenes Peptid ist, das eine Anzahl n von 6 bis 15 Aminosäuren enthält und das wenigstens ein Prolin an den Positionen 2 bis n-2 und/oder ein Histidin an den Positionen 1 bis n-2 aufweist.

3. Verfahren nach einem der vorherigen Ansprüche, **dadurch gekennzeichnet, dass** der durch Massenspektrometrie erzeugten quantitativen Dosierung von Peptiden eine Trennung durch Chromatographie oder Elektrophorese der in Schritt a) erzeugten Peptide vorangeht.

4. Verfahren nach Anspruch 3, **dadurch gekennzeichnet, dass** die Trennung durch Chromatographie eine Trennung durch Umkehrphasenchromatographie einsetzt.

5. Verfahren nach einem der vorherigen Ansprüche, **dadurch gekennzeichnet, dass** die durch Massenspektrometrie erzeugte quantitative Dosierung von Peptiden durch ein Massenspektrometer bewirkt wird, das einen Triple-Quadrupol umfasst.

6. Verfahren nach einem der vorherigen Ansprüche, **dadurch gekennzeichnet, dass** die durch Massenspektrometrie erzeugte quantitative Dosierung von Peptiden durch ein Massenspektrometer bewirkt wird, das eine lonenfalle umfasst.

7. Verfahren nach einem der vorherigen Ansprüche, **dadurch gekennzeichnet, dass** die Behandlung der Probe durch Verdau mit einem Proteaseenzym durchgeführt wird, beispielsweise Trypsin.

8. Verfahren nach einem der vorherigen Ansprüche, **dadurch gekennzeichnet, dass** in Schritt v) die Intensität des durch wenigstens einen Teil der lonenfragmente der zweiten Generation induzierten Stroms als eine Funktion der Zeit nachgewiesen wird und das über eine gegebene Periode erhaltene Signal in ein Massenspektrum unterschiedlicher Ionen als Funktion ihrer Masse m/z zerlegt wird, um einen Massenpeak in Bezug auf jedes der nachgewiesenen Ionen der zweiten Generation, das in der gegebenen Periode vorkommt, zu erhalten, und das dem Strom wenigstens eines ausgewählten Ions der zweiten Generation entsprechende Signal wieder zusammengesetzt wird und die Intensität des entsprechenden gemessenen Stroms die in Schritt vi) ausgewählte quantitative Messung ist.

9. Verfahren nach Anspruch 8, **dadurch gekennzeichnet, dass** in Schritt vi) die mit dem lonenfragment der zweiten Generation verbundene quantitative Messung ausgewählt wird, welche den stärksten Peak m/z in der gegebenen Periode aufweist.

10. Verfahren nach Anspruch 8 oder 9, **dadurch gekennzeichnet, dass** in Schritt vi) die Zuordnung auf Grundlage der Summe wenigstens zweier quantitativer Messungen erfolgt, wobei jede mit lonenfragmenten der zweiten Generation in Verbindung steht, welche in der gegebenen Periode die stärksten Peaks m/z aufweisen.

11. Verfahren nach einem der vorherigen Ansprüche, **dadurch gekennzeichnet, dass** die in Schritt vi) durchgeführte Zuordnung mittels einer Kalibrierungskurve erfolgt.

12. Verfahren nach einem der Ansprüche 1 bis 10, **dadurch gekennzeichnet, dass** die Zuordnung in Schritt vi) durch interne Kalibrierung mit einem schweren Peptid erfolgt.

## Claims

1. A method for the quantitative detection of a target protein in a sample, comprising the following steps:
a) treatment of the sample in order to generate peptides,
b) quantitative assaying of at least one proteotypic peptide generated from the target protein, via a mass spectrometry technique in which:
i) the proteotypic peptide is ionized to give precursor ions which are filtered according to their mass m/z, and a given precursor ion having a mass (m/z)₁ is selected according to the target protein sought,
ii) the selected precursor ion is fragmented into first-generation fragment ions,
iii) the first-generation fragment ions generated are filtered according to their mass m/z, and a given first-generation fragment ion having a mass (m/z)₂ is selected according to the target protein sought,
iv) the selected first-generation fragment ion is fragmented into second-generation fragment ions,
v) at least a part of the second-generation fragment ions are detected so as to give a series of quantitative measurements,
vi) at least one quantitative measurement associated with a second-generation ion is selected, and correlated to the amount of proteotypic peptide generated and to the amount of target protein present in the sample,
**characterized in that** the selected first-generation fragment ion having a mass (m/z)₂ is a doubly-charged peptide which has a proline and/or a histidine in position 1.

2. The method as claimed in claim 1, **characterized in that** the selected precursor ion having a mass (m/z)₁ is a doubly-charged peptide which contains a number n of from 6 to 15 amino acids, and which has at least one proline at positions 2 to n-2 and/or one histidine at positions 1 to n-2.

3. The method as claimed in one of the preceding claims, **characterized in that** the quantitative assaying of the generated peptides by mass spectrometry is preceded by a separation by chromatography or electrophoresis of the peptides generated in step a).

4. The method as claimed in claim 3, **characterized in that** the separation by chromatography uses a separation by reverse-phase chromatography.

5. The method as claimed in one of the preceding claims, **characterized in that** the quantitative assaying of the generated peptides by mass spectrometry is carried out with a mass spectrometer comprising a triple quadrupole.

6. The method as claimed in one of the preceding claims, **characterized in that** the quantitative assaying of the generated peptides by mass spectrometry is carried out with a mass spectrometer comprising an ion trap.

7. The method as claimed in one of the preceding claims, **characterized in that** the treatment of the sample is carried out by digestion with a protease enzyme, for example trypsin.

8. The method as claimed in one of the preceding claims, **characterized in that**, in step v), the intensity of the current induced by at least a part of the second-generation fragment ions is detected as a function of time, and the signal obtained over a given period is broken down into a mass spectrum of the various ions present according to their mass m/z, so as to obtain a mass peak associated with each of the second-generation ions detected present over the given period, and the signal corresponding to the current of at least one second-generation ion selected is recomposed, and the intensity of the corresponding current measured is the quantitative measurement selected in step vi).

9. The method as claimed in claim 8, **characterized in that**, in step vi), the quantitative measurement associated with the second-generation fragment ion having the most intense peak m/z over the given period is selected.

10. The method as claimed in claim 8 or 9, **characterized in that**, in step vi), the correlation is performed on the basis of the sum of at least two quantitative measurements, each being associated with the second-generation fragment ions having the most intense peaks m/z over the given period.

11. The method as claimed in one of the preceding claims, **characterized in that** the correlation carried out in step vi) is performed by means of a calibration curve.

12. The method as claimed in one of claims 1 to 10, **characterized in that** the correlation carried out in step vi) is performed by internal calibration with a heavy peptide.


### SEQUENCE LISTING

<110> bioMérieux Université Claude Bernard Lyon 1 Centre National de la Recherche Scientifique
<120> Nouveau procédé de quantification de protéines par spectrométrie de masse
<130> 14619FR35LSA/VF
<150> FR09 53576
   <151> 2009-05-29
<160> 54
<170> PatentIn version 3.3
<210> 1
   <211> 7
   <212> PRT
   <213> artificial sequence
<220>
   <223> OATP2B1
<400> 1
   Val Leu Leu Gln Thr Leu Arg
   1 5
<210> 2
   <211> 6
   <212> PRT
   <213> ARTIFICIAL SEQUENCE
<220>
   <223> OATP2B1
<400> 2
   Leu Leu Gln Thr Leu Arg
   1 5
<210> 3
   <211> 5
   <212> PRT
   <213> artificial sequence
<220>
   <223> OATP2B1
<400> 3
   Leu Gln Thr Leu Arg
   1 5
<210> 4
   <211> 16
   <212> PRT
   <213> artificial sequence
<220>
   <223> IFN-alpha4
<400> 4
   His Asp Phe Gly Phe Pro Gln Glu Glu Phe Gly Asn Gln Phe Gln Lys
   1 5 10 15
<210> 5
   <211> 15
   <212> PRT
   <213> artificial sequence
<220>
   <223> IFN-alpha4
<400> 5
   Asp Phe Gly Phe Pro Gln Glu Glu Phe Gly Asn Gln Phe Gln Lys
   1 5 10 15
<210> 6
   <211> 10
   <212> PRT
   <213> artificial sequence
<220>
   <223> IFN-alpha4
<400> 6
   Tyr Ser Pro Cys Ala Trp Glu Val Val Arg
   1 5 10
<210> 7
   <211> 8
   <212> PRT
   <213> artificial sequence
<220>
   <223> IFN-alpha4
<400> 7
   Pro Cys Ala Trp Glu Val Val Arg
   1 5
<210> 8
   <211> 14
   <212> PRT
   <213> artificial sequence
<220>
   <223> Plastine-1
<400> 8
   Gln Phe Val Thr Pro Ala Asp Val Val Ser Gly Asn Pro Lys
   1 5 10
<210> 9
   <211> 10
   <212> PRT
   <213> artificial sequence
<220>
   <223> Plastine-1
<400> 9
   Glu Ala Ser Leu Pro Leu Pro Gly Tyr Lys
   1 5 10
<210> 10
   <211> 9
   <212> PRT
   <213> artificial sequence
<220>
   <223> Plastine-1
<400> 10
   Leu Ser Pro Glu Glu Leu Leu Leu Arg
   1 5
<210> 11
   <211> 9
   <212> PRT
   <213> artificial sequence
<220>
   <223> Ezrin
<400> 11
   Ile Gly Phe Pro Trp Ser Glu Ile Arg
   1 5
<210> 12
   <211> 8
   <212> PRT
   <213> artificial sequence
<220>
   <223> Aminoacylase 1
<400> 12
   Val Ala Pro Asp Val Asp Phe Lys
   1 5
<210> 13
   <211> 11
   <212> PRT
   <213> artificial sequence
<220>
   <223> L-FABP
<400> 13
   Ala Ile Gly Leu Pro Glu Glu Leu Ile Gln Lys
   1 5 10
<210> 14
   <211> 17
   <212> PRT
   <213> artificial sequence
<220>
   <223> Protéine Disulfide-isomérase
<400> 14
   His Asn Gln Leu Pro Leu Val Ile Glu Phe Thr Glu Gln Thr Ala Pro
   1 5 10 15
   Lys
<210> 15
   <211> 13
   <212> PRT
   <213> artificial sequence
<220>
   <223> Protéine Disulfide-isomérase
<400> 15
   Asp Ala Pro Glu Glu Glu Asp His Val Leu Val Leu Arg
   1 5 10
<210> 16
   <211> 12
   <212> PRT
   <213> artificial sequence
<220>
   <223> Protéine Disulfide-isomérase
<400> 16
   Tyr Lys Pro Glu Ser Glu Glu Leu Thr Ala Glu Arg
   1 5 10
<210> 17
   <211> 9
   <212> PRT
   <213> artificial sequence
<220>
   <223> Protéine Disulfide-isomérase
<400> 17
   Thr His Ile Leu Leu Phe Leu Pro Lys
   1 5
<210> 18
   <211> 8
   <212> PRT
   <213> artificial sequence
<220>
   <223> Protéine Disulfide-isomérase
<400> 18
   Gln Leu Ala Pro Ile Trp Asp Lys
   1 5
<210> 19
   <211> 8
   <212> PRT
   <213> artificial sequence
<220>
   <223> Protéine Disulfide-isomérase
<400> 19
   Val His Ser Phe Pro Thr Leu Lys
   1               5
<210> 20
   <211> 8
   <212> PRT
   <213> artificial sequence
<220>
   <223> Protéine Disulfide-isomérase
<400> 20
   Phe Phe Pro Ala Ser Ala Asp Arg
   1               5
<210> 21
   <211> 8
   <212> PRT
   <213> artificial sequence
<220>
   <223> Protéine Disulfide-isomérase
<400> 21
   Ala Leu Ala Pro Glu Tyr Ala Lys
   1               5
<210> 22
   <211> 7
   <212> PRT
   <213> artificial sequence
<220>
   <223> Protéine Disulfide-isomérase
<400> 22
   Glu Glu Cys Pro Ala Val Arg
   1               5
<210> 23
   <211> 18
   <212> PRT
   <213> artificial sequence
<220>
   <223> Keratin, type 1 cytoskeletal 20
<400> 23
   Ser Leu Ser Ser Ser Leu Gln Ala Pro Val Val Ser Thr Val Gly Met
   1 5 10 15
   Gln Arg
<210> 24
   <211> 11
   <212> PRT
   <213> artificial sequence
<220>
   <223> Keratine, type 1 cytoskeletal 20
<400> 24
   Glu Ser Leu Glu His Thr Leu Glu Glu Thr Lys
   1 5 10
<210> 25
   <211> 14
   <212> PRT
   <213> artificial sequence
<220>
   <223> Keratin, type 1 cytoskeletal 20
<400> 25
   Leu Gly Thr Thr Pro Ser Val Tyr Gly Gly Ala Gly Gly Arg
   1 5 10
<210> 26
   <211> 9
   <212> PRT
   <213> artificial sequence
<220>
   <223> Keratin, type 1 cytoskeletal 20
<400> 26
   Gln Trp Tyr Glu Thr Asn Ala Pro Arg
   1 5
<210> 27
   <211> 9
   <212> PRT
   <213> artificial sequence
<220>
   <223> Keratin, type 1 cytoskeletal 20
<400> 27
   Val Phe Asp Asp Leu Thr Leu His Lys
   1 5
<210> 28
   <211> 9
   <212> PRT
   <213> artificial sequence
<220>
   <223> 14-3-3 protéine sigma
<400> 28
   Glu Met Pro Pro Thr Asn Pro Ile Arg
   1 5
<210> 29
   <211> 9
   <212> PRT
   <213> artificial sequence
<220>
   <223> Protéine S100-A11 Calgizarin
<400> 29
   Ile Ser Ser Pro Thr Glu Thr Glu Arg
   1 5
<210> 30
   <211> 7
   <212> PRT
   <213> artificial sequence
<220>
   <223> Protéine S100-A11 Calgizarin
<400> 30
   Asp Pro Gly Val Leu Asp Arg
   1 5
<210> 31
   <211> 10
   <212> PRT
   <213> artificial sequence
<220>
   <223> Plastine-1
<400> 31
   Pro Ala Asp Val Val Ser Gly Asn Pro Lys
   1 5 10
<210> 32
   <211> 6
   <212> PRT
   <213> artificial sequence
<220>
   <223> Plastine-1
<400> 32
   Pro Leu Pro Gly Tyr Lys
   1 5
<210> 33
   <211> 7
   <212> PRT
   <213> artificial sequence
<220>
   <223> Plastine-1
<400> 33
   Pro Glu Glu Leu Leu Leu Arg
   1 5
<210> 34
   <211> 6
   <212> PRT
   <213> artificial sequence
<220>
   <223> Ezrin
<400> 34
   Pro Trp Ser Glu Ile Arg
   1 5
<210> 35
   <211> 6
   <212> PRT
   <213> artificial sequence
<220>
   <223> Aminoacylase 1
<400> 35
   Pro Asp Val Asp Phe Lys
   1 5
<210> 36
   <211> 7
   <212> PRT
   <213> artificial sequence
<220>
   <223> L-FABP
<400> 36
   Pro Glu Glu Leu Ile Gln Lys
   1 5
<210> 37
   <211> 11
   <212> PRT
   <213> artificial sequence
<220>
   <223> Protéine Disulfide-isomérase
<400> 37
   Pro Glu Glu Glu Asp His Val Leu Val Leu Arg
   1 5 10
<210> 38
   <211> 10
   <212> PRT
   <213> artificial sequence
<220>
   <223> Protéine Disulfide-isomérase
<400> 38
   Pro Glu Ser Glu Glu Leu Thr Ala Glu Arg
   1 5 10
<210> 39
   <211> 8
   <212> PRT
   <213> artificial sequence
<220>
   <223> Protéine Disulfide-isomérase
<400> 39
   His Ile Leu Leu Phe Leu Pro Lys
   1 5
<210> 40
   <211> 5
   <212> PRT
   <213> artificial sequence
<220>
   <223> Protéine Disulfide-isomérase
<400> 40
   Pro Ile Trp Asp Lys
   1 5
<210> 41
   <211> 7
   <212> PRT
   <213> artificial sequence
<220>
   <223> Protéine Disulfide-isomérase
<400> 41
   His Ser Phe Pro Thr Leu Lys
   1 5
<210> 42
   <211> 6
   <212> PRT
   <213> artificial sequence
<220>
   <223> Protéine Disulfide-isomérase
<400> 42
   Pro Ala Ser Ala Asp Arg
   1 5
<210> 43
   <211> 5
<212> PRT
   <213> artificial sequence
<220>
   <223> Protéine Disulfide-isomérase
<400> 43
   Pro Glu Tyr Ala Lys
   1 5
<210> 44
   <211> 4
   <212> PRT
   <213> artificial sequence
<220>
   <223> Protéine Disulfide-isomérase
<400> 44
   Pro Ala Val Arg
   1
<210> 45
   <211> 7
   <212> PRT
   <213> artificial sequence
<220>
   <223> Keratin, type 1 cytoskeletal 20
<400> 45
   His Thr Leu Glu Glu Thr Lys
   1 5
<210> 46
   <211> 10
   <212> PRT
   <213> artificial sequence
<220>
   <223> Keratin, type 1 cytoskeletal 20
<400> 46
   Pro Ser Val Tyr Gly Gly Ala Gly Gly Arg
   1 5 10
<210> 47
   <211> 7
   <212> PRT
   <213> artificial sequence
<220>
   <223> 14-3-3 protéine sigma
<400> 47
   Pro Pro Thr Asn Pro Ile Arg
   1 5
<210> 48
   <211> 6
   <212> PRT
   <213> artificial sequence
<220>
   <223> Protéine S100-A11 Calgizarin
<400> 48
   Pro Thr Glu Thr Glu Arg
   1 5
<210> 49
   <211> 6
   <212> PRT
   <213> artificial sequence
<220>
   <223> Protéine S100-A11 Calgizarin
<400> 49
   Pro Gly Val Leu Asp Arg
   1 5
<210> 50
   <211> 12
   <212> PRT
   <213> artificial sequence
<220>
   <223> PSA
<400> 50
   Leu Ser Glu Pro Ala Glu Leu Thr Asp Ala Val Lys
   1 5 10
<210> 51
   <211> 152
   <212> PRT
   <213> artificial sequence
<220>
   <223> Tp435
<400> 51
   Met Arg Gly Ser Ala Cys Val Ser Cys Thr Thr Val Cys Pro His Ala
   1 5 10 15
   Gly Lys Ala Lys Ala Glu Lys Val Glu Cys Ala Leu Lys Gly Gly Ile
   20 25 30
   Phe Arg Gly Thr Leu Pro Ala Ala Asp Cys Pro Gly Ile Asp Thr Thr
   35 40 45
   Val Thr Phe Asn Ala Asp Gly Thr Ala Gln Lys Val Glu Leu Ala Leu
   50 55 60
   Glu Lys Lys Ser Ala Pro Ser Pro Leu Thr Tyr Arg Gly Thr Trp Met
   65 70 75 80
   Val Arg Glu Asp Gly Ile Val Glu Leu Ser Leu Val Ser Ser Glu Gln
   85 90 95
   Ser Lys Ala Pro His Glu Lys Glu Leu Tyr Glu Leu Ile Asp Ser Asn
   100 105 110
   Ser Val Arg Tyr Met Gly Ala Pro Gly Ala Gly Lys Pro Ser Lys Glu
   115 120 125
   Met Ala Pro Phe Tyr Val Leu Lys Lys Thr Lys Lys Gly Ser Ser Lys
   130 135 140
   Tyr Lys Tyr His His His His His
   145 150
<210> 52
   <211> 428
   <212> PRT
   <213> artificial sequence
<220>
   <223> Tp574
<400> 52
   Met Arg Gly Ser Ala His His Glu Thr His Tyr Gly Tyr Ala Thr Leu
   1               5                   10                  15
   Ser Tyr Ala Asp Tyr Trp Ala Gly Glu Leu Gly Gln Ser Arg Asp Val
               20                  25                  30
   Leu Leu Ala Gly Asn Ala Glu Ala Asp Arg Ala Gly Asp Leu Asp Ala
           35                  40                  45
   Gly Met Phe Asp Ala Val Ser Arg Ala Thr His Gly His Gly Ala Phe
       50                  55                  60
   Arg Gln Gln Phe Gln Tyr Ala Val Glu Val Leu Gly Glu Lys Val Leu
   65                  70                  75                  80
   Ser Lys Gln Glu Thr Glu Asp Ser Arg Gly Arg Lys Lys Trp Glu Tyr
                   85                  90                  95
   Glu Thr Asp Pro Ser Val Thr Lys Met Val Arg Ala Ser Ala Ser Phe
               100                 105                 110
   Gln Asp Leu Gly Glu Asp Gly Glu Ile Lys Phe Glu Ala Val Glu Gly
           115                 120                 125
   Ala Val Ala Leu Ala Asp Arg Ala Ser Ser Phe Met Val Asp Ser Glu
       130                 135                 140
   Glu Tyr Lys Ile Thr Asn Val Lys Val His Gly Met Lys Phe Val Pro
   145                 150                 155                 160
   Val Ala Val Pro His Glu Leu Lys Gly Ile Ala Lys Glu Lys Phe His
                   165                 170                 175
   Phe Val Glu Asp Ser Arg Val Thr Glu Asn Thr Asn Gly Leu Lys Thr
               180                 185                 190
   Met Leu Thr Glu Asp Ser Phe Ser Ala Arg Lys Val Ser Ser Met Glu
           195                 200                 205
   Ser Pro His Asp Leu Val Val Asp Thr Val Gly Thr Gly Tyr His Ser
       210                 215                 220
   Arg Phe Gly Ser Asp Ala Glu Ala Ser Val Met Leu Lys Arg Ala Asp
   225                 230                 235                 240
   Gly Ser Glu Leu Ser His Arg Glu Phe Ile Asp Tyr Val Met Asn Phe
   245 250 255
   Asn Thr Val Arg Tyr Asp Tyr Tyr Gly Asp Asp Ala Ser Tyr Thr Asn
   260 265 270
   Leu Met Ala Ser Tyr Gly Thr Lys His Ser Ala Asp Ser Trp Trp Lys
   275 280 285
   Thr Gly Arg Val Pro Arg Ile Ser Cys Gly Ile Asn Tyr Gly Phe Asp
   290 295 300
   Arg Phe Lys Gly Ser Gly Pro Gly Tyr Tyr Arg Leu Thr Leu Ile Ala
   305 310 315 320
   Asn Gly Tyr Arg Asp Val Val Ala Asp Val Arg Phe Leu Pro Lys Tyr
   325 330 335
   Glu Gly Asn Ile Asp Ile Gly Leu Lys Gly Lys Val Leu Thr Ile Gly
   340 345 350
   Gly Ala Asp Ala Glu Thr Leu Met Asp Ala Ala Val Asp Val Phe Ala
   355 360 365
   Asp Gly Gln Pro Lys Leu Val Ser Asp Gln Ala Val Ser Leu Gly Gln
   370 375 380
   Asn Val Leu Ser Ala Asp Phe Thr Pro Gly Thr Glu Tyr Thr Val Glu
   385 390 395 400
   Val Arg Phe Lys Glu Phe Gly Ser Val Arg Ala Lys Val Val Ala Gln
   405 410 415
   Ser Ser Lys Tyr Lys Thr His His His His His His
   420 425
<210> 53
   <211> 9
   <212> PRT
   <213> artificial sequence
<220>
   <223> Tp435
<400> 53
   Ser Ala Pro Ser Pro Leu Thr Tyr Arg
   1 5
<210> 54
   <211> 11
   <212> PRT
   <213> artificial sequence
<220>
   <223> Tp574
<400> 54
   Phe Val Pro Val Ala Val Pro His Glu Leu Lys
   1               5                   10